# EUROPEAN PATENT SPECIFICATION

(11) **EP 1 410 017 B1**
(45) Date of publication and mention of the grant of the patent: **19.08.2009**
(21) Application number: 02713522.7
(22) Date of filing: 31.01.2002
(51) Int. Cl.: G01N 33/50, G01N 33/566

(54) **GAP JUNCTION PERMEABILITY ASSAY**
"GAP JUNCTION" PERMEABILITÄTS ASSAY
ANALYSE DE PERMEABILITE DES JONCTIONS LACUNAIRES

(30) Priority: 06.02.2001 US 266641 P; 08.09.2001 GB 0119438
(43) Date of publication of application: 21.04.2004
(73) Proprietor: Aventis Pharmaceuticals Inc., Bridgewater, New Jersey 08807 (US)
(72) Inventor: BARBIER, Ann, J., La Jolla, CA 92037 (US); BARON, Bruce, M., Westfield, NJ 07090 (US)
(74) Representative: Löwrick, Oliver
(86) International application number: PCT/US2002/002954
(87) International publication number: WO 2002/063298

(56) References cited:
- WO-A-00/13651
- WO-A-99/26584
- BARBIER ANN J ET AL: "Development of a prototype reporter-based assay for the evaluation of gap junctional intercellular communication." BIOTECHNOLOGY LETTERS, vol. 23, no. 19, October 2001 (2001-10), pages 1559-1563, XP009005715 ISSN: 0141-5492
- ANDRADE-ROZENTAL A F ET AL: "Gap junctions: The "kiss of death" and the "kiss of life"." BRAIN RESEARCH REVIEWS, vol. 32, no. 1, April 2000 (2000-04), pages 308-315, XP002231074 ISSN: 0165-0173
- SANDBERG K ET AL: "ANGIOTENSIN II-INDUCED CALCIUM MOBILIZATION IN OOCYTES BY SIGNAL TRANSFER THROUGH GAP JUNCTIONS" SCIENCE (WASHINGTON D C), vol. 249, no. 4966, 1990, pages 298-301, XP001097272 ISSN: 0036-8075
- MURRAY S A ET AL: "HORMONE INDUCED INTER CELLULAR SIGNAL TRANSFER DISSOCIATES CYCLIC AMP DEPENDENT PROTEIN KINASE" JOURNAL OF CELL BIOLOGY, vol. 98, no. 5, 1984, pages 1710-1719, XP009005845 ISSN: 0021-9525
- KUMAR NALIN M ET AL: "The gap junction communication channel." CELL, vol. 84, no. 3, 1996, pages 381-388, XP002231075 ISSN: 0092-8674
- GEORGE C H ET AL: "Rapid determination of gap junction formation using HeLa cells microinjected with cDNAs encoding wild-type and chimeric connexins." BIOCHEMICAL AND BIOPHYSICAL RESEARCH COMMUNICATIONS. UNITED STATES 29 JUN 1998, vol. 247, no. 3, 29 June 1998 (1998-06-29), pages 785-789, XP002231076 ISSN: 0006-291X

## Description

### Background on Invention

Intercellular communication is important for coordinating the behavior of cell assemblies and plays an important role in cell development, differentiation, and the normal functioning of organs such as the heart and brain. A large variety of cell types within an organism contain specialized proteins suitable for intercellular communication, characterized as gap junction channels, reviewed in Dermietzel, R., Brain Research Reviews, 26, 176-183, 1998 and Kumar, N. and Gilula, N., Cell, 84, 381-388, 1996. Gap junction channels are composed of a family of proteins, denoted as connexins, which are believed to assemble as hexameric complexes on the cell surface. The resulting complexes are called connexons and, when paired with a complementary connexon on another cell, can form a continuous channel between the two cells called a gap junction. In certain experimental paradigms, an unpaired connexon also shows ion permeability and is called a hemichannel.

Connexin nomenclature is based on the molecular weight of the particular protein. For example, "Cx43" refers to a 43 kDa molecular weight connexin protein which is expressed in liver, heart and brain. The size of the connexin family is increasing due to molecular cloning techniques. At present, 11 human and 13 rodent connexins have been described at the molecular level. Some of the connexins have polymorphisms in their coding sequence which may affect their conductance properties or their ability to associate into gap junctions. Furthermore, mutations in certain connexins are being identified with particular diseases. For example, Cx32 mutants have been linked to a peripheral neuropathy, Charcot Marie Tooth disease. Connexin mutations are also associated with deafness and with skin diseases such as palmoplantar keratoderma, with numerous aberrent Cx26 and Cx31 mutations identified. Mutations in Cx46, Cx50 and Cx43 have also been identified linked to cataract or heart malformations. Mutations in Cx37 have been linked to female infertility. Connexins are also associated with tumors, for example, Cx32 knockout mice show high incidences of spontaneous and chemically induced liver tumors. Transfection of connexin genes into tumorigenic cells restores normal growth. Studies with transgenic mice overexpressing mutant Cx32 in liver cells indicate that Cx32 plays a key role in liver regeneration after partial hepatectomy.

It is clear on such a background of molecular diversity of connexin species that there is a large number of combinatorial possibilities for the composition of gap junctions. There are 3 levels of complexity: connexin, connexon, and junction. A given tissue or cell type generally expresses multiple connexins. The connexins have been shown to assemble both into homomeric (composed of a single species) or heteromeric (composed of more than one connexin protein type) connexons. Finally, different species of homomeric connexons have been shown to form functional junctions. This range of possibilities is reduced to some extent by the tissue- and cell-specific expression patterns of the connexin family members and the observation that certain combinations of connexons are unable to form functional junctions. Nevertheless, it is expected that there will be a considerable molecular diversity of junctional structures which will be differentiated both pharmacologically and physiologically leading to specific therapeutic targets.

In the brain, junctional propagation of calcium transients is thought to underlie the calcium waves seen in spreading depression and after application of glutamate (Venance, L. et al., J. Neuroscience, 17, 1981-1992, 1997). Injury propagation mediated by gap junctions has been seen in glial cell cultures exposed to a variety of toxic insults. Cells sensitive to the insult can transmit the "death signal" to normally insensitive cells via junctional communication (Lin, J., et al., Nature Neuroscience, 1, 494-500, 1998). These authors suggest that this phenomenon provides a model for infarct enlargement following cerebral ischemia. Junctional coupling of myocardial cells contributes to impulse propagation in the heart. Inhibition of cardiac junctional transmission produces arrhythmia whereas facilitation can relieve conduction abnormalities (Dhein, S. et al., Naunyn-Schmiedeberg's Arch. Pharmacol., 350, 174-184, 1994). Morphogenetic signaling such as the organized migration of neuroblasts, differentiation of hepatocytes, and de-differentiation during tumor formation have each been hypothesized to be the result of alterations in the level of junctional coupling, reviewed in reviews cited above and Zhu, D. et al., Proc Natl Acad. Sci. USA, 89, 10218-10221, 1992.

One of the features that distinguishes connexin-mediated conductance from that of conventional ion channels is the larger pore radius characteristic of gap junctions. Indeed, gap junctions are known to be permeable to a diverse set of molecules up to 1 kDa in size allowing passage of ions, second messengers, and metabolites (e.g. cyclic AMP, inositol trisphosphate, calcium and potassium; see review articles cited above).

Existing assays for monitoring junctional permeability utilize the pore radius and relative lack of specificity. These assays are distinguished from the present invention by the means by which the cells are loaded with tracer substance. Generally, these methods involve the use of molecules which can be directly visualized, such as fluorescent dyes, or detected after enzymatic amplification, such as biotin-tagged tracers. In the scrape-loading paradigm, cells in monolayer culture are locally-damaged by scratching the surface with a stylus. A mixture of fluorescent dyes, e.g. lucifer yellow and rhodamine-coupled dextran, are applied to the monolayer for a period of time and then the cells are washed, fixed, and examined under the microscope. Both dyes enter the monolayer in the vicinity of the scratch and fill the cytoplasm of the injured cells. The lucifer yellow is able to diffuse through the gap junctions, staining neighboring cells which are coupled through gap junctions. In contrast, the rhodamine dextran is a large polymer unable to penetrate gap junctions and labels only the damaged cells in the vicinity of the scratch. The number of luciferase-positive cells relative to the number of rhodamine-positive cells provides an index of coupling through gap junctions. A similar method involves dye introduction into single cells via microinjection of tracers such as neurobiotin. Another variant uses cell permeable precursors of fluorescent dyes, e.g. carboxymethylfluorescein, to load cells. After cleavage of the dye by cytosolic esterases a membrane-impermeable dye is produced, e.g. carboxyfluoroscein. The cleaved product retains gap junctional permeability and can serve as a marker for intercellular coupling. Exposure of a small region of the loaded cells to high intensity light permanently inactivates the fluorophore by a process known as fluorescent photobleaching. It is then possible to monitor the bleached region for recovery of the fluorescent signal over time since this represents refilling by dye molecules located in coupled cells. The methods described above have several limitations in that they require mechanical intervention, are generally of low sensitivity, are difficult to quantitate, and are not amenable to automation. These features limit their use in situations in which there is a low extent of coupling or in which numerous measurements must be taken as in the process of discovery of agents which modulate junctional transmission, El Fouly, M.H. et al., Exp. Cell Res., 168, 519-526, 1987; Giuame, C. et al., Proc. Natl. Acad. Sci. USA, 88, 5577-5581,1991.

Goldberg et al. have described a technique which circumvents some of these limitations by pre-loading two fluorescent dyes (Goldberg et al., Biotechniques, 18(3): 490497, 1995). In this method, the "sender" cells containing connexins are loaded with a junction-permeable dye (calcein-AM) and a membrane marker dye DiI (1,1'-dioctadecyl-3,3,3',3'-tetramethylindocarocyanine perchlorate). The cells are then washed, suspended, and mixed with an unloaded connexin-containing cell population. Junctional transmission is detected by measuring the number of cells exhibiting calcein fluorescence in the absence of Dil fluorescence. While dye introduction in this method is straightforward, the disadvantage is that either human intervention or sophisticated image processing methods are needed to identify and quantify cells exhibiting only calcein fluorescence.

The prior art also teaches metabolic defect assay, wherein a first cell has a defect in one part of a necessary metabolic pathway but is capable of synthesizing a small metabolite capable of moving through a gap junction, the second cell has a different defect in the same necessary metabolic pathway, but corrected by the small metabolite made by the first cell. When the two cells are co-cultured, and a functional gap junction forms between the two cells, the small metabolite moves into the second cell, thereby maintaining the viability of the second cell.

ANDRADE-ROZENTAL A.F. ET AL (BRAIN RESEARCH REVIEWS, vol. 32, no. 1, April 2000 (2000-04), pages 308-315) demonstrates bystander effects in transfected cells expressing herpes simplex-thymidine kinase (HSV-tk) and Cx37, treated with ganciclovir (corresponding to the external stimulus). Apoptosis is induced in neighboring, untransfected cells. The extent and sensitivity to cell death is a function of connexin expression.

SANDBERG K. ET AL. (SCIENCE WASHINGTON D.C.), vol. 249, no. 4966, 1990, pages 298-301) describes angiotensin II-induced calcium mobilisation in oocytes by signal transfer through gap junctions. Aequorin injected into cells is used as the calcium indicator. Experiments are performed with mixed cell cultures of follicular cells and oocytes. It is noted that since aequorin is too large to pass through gap junctions, the results of Sandberg et al. suggest that endogenous receptor located on follicular cells communicate with the oocyte by generating second messenger molecules that diffuse through gap junctions and mobilise stored Ca2+ (see page 300, first column).

MURRAY S.A. ET AL. (JOURNAL OF CELL BIOLOGY, vol. 98, no. 5,1984, pages 1710-1719) pertains to signal transfer between co-cultured adrenocortical tumour cells (Y-1) and porcine ovarian granulosa cells. Stimulation of the co-cultured cells with either ATCH or FSH (follicle stimulating hormone) leads to a messenger response, i.e. the dissociation of cAMP-dependent protein kinase in adjacent cells, connected to the stimulated cells by gap junctions (see passages cited in the search report). Even though the nature of the signalling molecule is not specifically determined in Murray et al., cAMP appears to be a likely candidate.

We describe here a gap junction coupling assay which is highly sensitive, quantitative and can be readily automated for screening of large compound libraries to identify compounds which modulate junctional transmission.

### Summary of the Invention

The assay of the present invention provides a convenient, rapid, reproducible method for investigating gap junction intercellular communication (hereafter abbreviated GJIC), and more specifically for screening for compounds that affect GJIC. Alternatively, the invention is also directed to a method of identifying novel connexins capable of forming functional gap junctions. An important feature of the present invention is the use of two different cell populations, one cell type is a sender cell line capable of generating an endogenous messenger response, and a second cell type is a receiver cell type capable of generating a reporter response in response to the endogenous messenger response, where the endogenous messenger response is transferred to the receive cell through a functional gap junction, or connexon, formed by the connexins on the cell surfaces of the sender and receiver cells. The principle of the assays is the separation of the generation of a signal, i.e. endogenous messenger response, from its detection as a reporter response which may be activation of expression of a reporter gene, in two different cell types which requires an active gap junction formed by a connexon.

### Detailed Description of the Invention

The present method of the invention is to identify a test compound capable of altering gap junction function, comprising
(1) adding a test compound to an assay reaction wherein the assay reaction comprises a sender cell and a receiver cell, wherein
   (a) the sender cell (1) expresses a first connexin on the cell surface which forms a first connexon and (2) is capable of generating an endogenous messenger response in response to an exogenous stimulus;
   (b) the receiver cell (1) expresses a second connexin on the cell surface which forms a second connexon (2) is not capable of generating an endogenous messenger response to an exogenous stimulus, and (3) is capable of generating a reporter response in response to the endogenous messenger response generated by the sender cell;
   (c) the first connexon and the second connexon form a gap junction;
(2) adding an exogenous stimulus to the assay reaction; and
(3) detecting the reporter response;
   wherein the reporter response is transcription of a second reporter gene linked to a promoter-reporter construct containing a cAMP responsive element (CRE), or a reporter gene linked to a promoter-reporter construct containing an SRE or TRE response element.

A 'test compound' is any organic or inorganic molecule, or complex of molecules. Test compounds may be naturally-occurring, or synthesized molecules. Preferred test compounds are organic molecules. More preferred test compounds are organic molecules of less than about 5,000 Da, and still more preferred test compounds are organic molecules of less than about 2,000 Da.

'Capable of altering' refers to the ability of a test compound to affect, whether directly or indirectly, properties of the gap junction from those properties in the absence of the test compound. 'Altering' includes changing gap junction properties such as permitting larger molecules to pass through, as well as limiting the size of molecules capable of passing through the gap junction. 'Altering' also includes complete inhibition of any molecule passing through the gap junction. 'Altering' also includes changing a defective gap junction to restore the gap junction to a normal function. Altering also includes selectivity for changing the properties of certain gap junction connexon and not others, or the ability to affect the properties of other connexons to a lesser degree. A test compound directly affects the gap junction when the test compound binds directly to the connexon. A test compound indirectly affects the gap junction when the test compound bind to or interacts with a cellular component, such as a transcription factor, which because of that binding or interaction, alters the gap junction.

A 'gap junction' is a type of junctional complex formed between adjacent cells and consist of an aggregated channel that directly links the interiors of neighboring cells. A gap junction results from two connexons each on a different cell, to join together to form a channel which permits generally small molecules to move from one cell into a second cell. These small molecules are typically up to about one to about two kDa in size, and are usually ions, second messengers such as cAMP, cGMP, calcium, inositol trisphosphate or cellular metabolites.

An 'assay reaction' is generally an aqueous environment, suitable for permitting cell-to-cell contact and contains a sender cell and a receiver cell. Preferred 'assay reactions' also comprise growth media suitable for supporting the continued cell growth and/or maintenance. It is preferred that the assay reaction contain more than one sender cell, or alternatively, more than one receiver cell. It is especially preferred that the assay reaction contain more than one receiver and more than one sender cell.

The selection of suitable receiver and sender cells is not limited to a particular cell type, nor is it required to be the same for both sender and receiver. Suitable sender or receiver cells comprise those obtained from animals including mammalian cell lines, particularly preferred cell lines are those isolated or cultured from human tissues. Cell lines may be grown in primary cultures or cell lines can be used. Sources of suitable cell lines include cell lines available from commercial sources, such as the American Type Culture Collection (ATCC). There is no requirement for the sender and receiver to be the same cell type though this will be the most common means of using this method.

A 'sender cell' (1) expresses a first connexin on the cell surface which forms a first connexon and (2) is capable of generating an endogenous messenger response in response to an exogenous stimulus.

A 'receiver cell' (1) expresses a second connexin on the cell surface which forms a second connexon, (2) is not capable of generating an endogenous messenger response to an exogenous stimulus , and (3) is capable of generating a reporter response in response to the endogenous messenger response generated by the sender cell.

'Exogenous' refers to something produced from or originating from outside the first cell, or due to external causes from the first cell, or alternatively, is introduced from or produced outside the first cell. An exogenous stimulus may be a peptide hormone, steroid, or other molecule produced by a different cell, or a small organic molecule.

'Endogenous' refers to something originating from within the first cell, or arises from internal causes within the first cell. An endogenous messenger response is typically cAMP, cGMP, calcium or inositol trisphosphate. Preferred endogenous messenger responses for use in the present invention are small molecules typically up to about one to about two kDa in size, and are usually ions, second messengers such as cAMP, cGMP, calcium, inositol trisphosphate or cellular metabolites. Alternatively, sugars, nucleotides, amino acids, fatty acids, small peptides, drugs and carcinogens may be used as the endogenous messenger response.

'Connexins' suitable for use in the present invention include naturally- and non-naturally-occurring connexins. Naturally occurring connexins include connexins with normal (i.e., wildtype) as well as mutant forms of connexins. Mutant forms of connexins include those naturally-occurring variants of wildtype connexins such as sequenced from humans or other species, as well as artificially-modified variant forms, which might include mouse-human chimeras, or a chemically-modified human Cx43. Connexins may be selected from those previously identified as connexins, as provided in Tables 1 (Human Connexins) and 2 (Rodent Connexins) below, or ortholog or homolog thereof, or another species connexin. Proteins which are putative connexins may also be used in the present invention. Identity as a putative connexin may be based on a high degree of sequence homology to connexins previously identified and demonstrated capable of forming connexons, preferably proteins with at least 70% sequence homology with a connexin, more preferably proteins with at least 80% sequence homology with a connexin, even more preferably proteins with at least 90% sequence homology with a connexin. In one aspect of the present invention, the assay described herein may be used to confirm identity of a putative connexin as a 'connexin', by demonstrating that the putative connexin is capable of forming a gap junction with a known connexin. The present invention may also be used to characterize with which other connexins a putative connexin is capable of forming gap junctions.

**Table 1. List of known human connexin nucleic acid sequences (partial clones and sequence variants included) and GeneBank Accession Numbers.**

| Name | Accession Number | Name | Accession Number |
|---|---|---|---|
| Cx26 | NM_004004 | Cx37 variant | AF139105 |
| Cx26 | M86849 | Cx37 variant | AF13904 |
| Cx26 | U43932 | Cx37 variant | AF139103 |
| Cx30 | NM_006783 | Cx37 variant | AF139102 |
| Cx30 | AJ005585 | Cx37 variant | AF139101 |
| Cx31 | AF052692 | Cx37 variant | AF139100 |
| Cx31 | AF099730 | Cx40 | NM_005266 |
| Cx31 | AJ004856 | Cx40 | AF151979 |
| Cx31.1 | AF052693 | Cx40 | L34954 |
| Cx31.1 | AF099731 | Cx43 | NM_000165 |
| Cx32 | NM_000166 | Cx43 | AF151980 |
| Cx32 | L47127 | Cx43 | U64573 |
| Cx36 | AB037509 | Cx43 | M65188 |
| Cx36 | AF153047 | Cx43 pseudogene | M65189 |
| Cx37 | NM_002060 | Cx43-related pseudogene | Z92542 |
| Cx37 | M96789 | Cx45 | NM_005497 |
| Cx37 | AF181620 | Cx50 | AF217524 |
| Cx37 polymorph | AF180815 | Cx50 | NM_005267 |

**Table 2. List of known rodent connexin nucleic acid sequences (partial clones and sequence variants included) and GeneBank Accession Numbers.**

| Name | Accession Number | Species | Name | Accession Number | Species |
|---|---|---|---|---|---|
| α3-Connexin (Cx46) | U44955 | Mouse | Cx32 | X84215 | Mouse |
| Cx26 | M81445 | Mouse | Cx32 | X84214 | Mouse |
| Cx26 | M81444 | Mouse | Cx32 | M81447 | Mouse |
| Cx26 | M63803 | Mouse | Cx32 | M81446 | Mouse |
| Cx30 | NM_008128 | Mouse | Cx32 | M63802 | Mouse |
| Cx30 | Z70023 | Mouse | Cx36 | AF016190 | Mouse |
| Cx30.3 | M91443 | Mouse | Cx37 | X57971 | Mouse |
| Cx31 | X63099 | Mouse | Cx40 | X61675 | Mouse |
| Cx31.1 | M91442 | Mouse | Cx43 | NM_010288 | Mouse |
| Cx31.1 | M91236 | Mouse | Cx43 | U17892 | Mouse |
| Cx32 | AJ271753 | Mouse | Cx43 | X62836 | Mouse |
| Cx43 | X61576 | Mouse | Cx33 | M76534 | Rat |
| Cx43 | M63801 | Mouse | Cx36 | Y16898 | Rat |
| Cx43 | L10388 | Mouse | Cx37 | M76532 | Rat |
| Cx43 | M61896 | Mouse | Cox40 | AF022136 | Rat |
| Cx43 | L10387 | Mouse | Cx40 | AF021806 | Rat |
| Cx45 | X63100 | Mouse | Cx40 | AF025765 | Rat |
| Cx50 | NM_008123 | Mouse | Cx40 | AH006192 | Rat |
| Cx50 | M91243 | Mouse | Cx40 | AF025767 | Rat |
| Cx57 | NM_010289 | Mouse | Cx40 | AF025766 | Rat |
| Cx57 | AJ010741 | Mouse | Cx40 | M83092 | Rat |
| Cox26 | X51615 | Rat | Cx40 | M76535 | Rat |
| Cx30 | AF170284 | Rat | Cx43 | NM_012S67 | Rat |
| Cx30.3 | X76168 | Rat | Cx43 | X06656 | Rat |
| Cx31 | M59936 | Rat | Cx43 | AH003191 | Rat |
| Cx32 | M23565 | Rat | Cx43 | L36950 | Rat |
| Cx32 | AH003192 | Rat | Cx43 | L36949 | Rat |
| Cx32 | L36875 | Rat | Cx46 | X57970 | Rat |
| Cx32 | L36876 | Rat | CXN-311 | M76533 | Rat |

| | | | | | |
|---|---|---|---|---|---|
| Mutant forms of mammalian connexins associated with diseases are preferred forms of mutant connexins. | | | | | |

In one embodiment of the present invention, the first and second connexin are selected from connexin family members which are known to form functional gap junctions. This would include a single connexin species which forms homomeric connexons or a mixture of connexins which results in a heteromeric connexon. The sender and receiver cell can have the same or different connexon composition with the requirement that the two connexons are able to form a functional gap junction. Thus, this method is applicable to both homotypic and heterotypic gap junctions. The connexin can be introduced by transfection and can be expressed transiently or stably integrated to produce a connexin-expressing cell line. Alternatively, the method is applicable to cells which express endogenous connexins and do not require transfection.

The connexins useful in the present invention may be normally expressed native connexins, which means that the cell is capable of expressing the particular connexin under ordinary cultured conditions. Native expression of the connexin may be constitutive, such as where the connexin is normally present on the cell surface of the cell in measurable amounts, or alternatively, expression of a native connexin may be induced under conditions known to promote expression of that connexin. Alternatively, the connexins may be non-native, and thus expressed under the control of a non-native promoter if the cell is transfected or transformed with an expression vector with DNA encoding the connexin linked to an expression vector. Transfection methods useful in generating transfected cells useful in the present invention include calcium phosphate transfection, using DEAE-Dextran, by electroporation, or alternatively, using cationic lipid reagents. Genes may be introduced using transduction by retroviral vectors or alternatively, by gene targeting by homologous recombination. In addition, the native connexin may be selectively altered by mutagenesis of cloned DNA using, for example, oligonucleotide-directed mutagenesis, by mutagenesis with degenerate oligonucleotides, by random mutagenesis by PCR, by linker-scanning mutagenesis of DNA, or by directed mutagenesis using the polymerase chain reaction. In addition, a connexin encoding either a native or non-native connexin protein may be created de novo by gene synthesis using mutually priming long oligonucleotides. Alternatively, the connexin may be expressed in the cell as result of a non-native promoter replacing the native promoter, such as by using homologous recombination to replace a native promoter with another promoter. Techniques and reagents suitable for transforming or transfecting cells in order to obtain non-native protein expression are well known to those skilled in the art, see for example, Current Protocols in Molecular Biology, John Wiley & Sons. Non-native connexins, such as a human connexin, may be expressed in a non-human cell such as CHO cell, murine, or other cell derived from a non-human species.

A'connexon' which forms a gap junction includes homomeric, composed of a single species of connexin, and heteromeric, composed of more than one connexin protein type. Heteromeric gap channels may have different permeability and regulatory properties than homomeric ones, and may provide numerous additional options for regulating the types of molecules that pass through its gap junction channel. A connexon is formed by the aggregation of six protein subunits known as connexins, which are folded in the plasma membrane in the approximate shape of an 'M'. The amino and carboxyl termini project into the cytoplasm, while the remainder of the connexin traverses the plasma membrane four times. The third membrane-spanning domain of connexin contains a high proportion of hydrophilic amino acids, and is thought to line the interior of the channel. The four membrane-spanning domains and the extracellular loops are highly conserved between the many different connexins that have been cloned, with less homology between the cytoplasmic regions. Some connexins are phosphorylated on the carboxyl tail. The connexin may be of the same, such as the same class of connexin for example, Cx32, or different species, such as each from a different class, for example, Cx32 and Cx43, on the sender or receiver cell. The connexin may also be the same class of connexin, but selected from different animal or mammalian species, such as mouse Cx32 and human Cx32. The connexins may be naturally-occurring connexin, or a mutant form associated with a disease state. The connexin may be a non-naturally-occurring connexin, a chimera where one or more amino acids in one or more domains of the connexin is substituted with amino acids of a corresponding domain in a different connexin, is substituted with amino acids of a similar charge, polarity, or size, or is deleted. Alternatively, a phosphorylated-region of a carboxy tail of phosphorylated connexin may be substituted or added to a connexin which is not normally phosphorylated. In the preferred embodiment, the connexon formed on the surface of the first and second cells forms a functional gap junction which permits intercellular transport of small molecules such as ions, sugars, nucleotides, amino acids, fatty acids, small peptides, drugs and carcinogens. It is also understood that the gap junction formed by the first and second connexon is dysfunctional or defective, such that transport of small molecules is impaired or even prevented relative to other gap junctions.

In an alternative embodiment of the invention, the purpose of the assay may be to identify test compounds that fix defective, or mutant, forms of gap junctions. Such an assay may be particularly useful for identifying test compounds capable of restoring normal function of disease-related mutant gap junctions. In such an embodiment, connexons form a defective gap junction and are ordinarily incapable of intercellular transfer of small molecules, or have impaired intercellular transfer. However, if the test compound is capable of altering the deficient gap junction, then a functional gap junction would form between the connexons, and the second messenger signal would be able to elicit the detectable response in the receiver cell. Such an assay would be useful to screen for 'corrective' test compounds, rather than 'inhibitory' compounds.

In another embodiment of the present invention, the purpose of the assay may be to identify test compounds that affect expression of a connexin. For example, physiological, pharmacological and dietary factors alter the expression of connexins, often in a cell-specific manner. Expression of Cx32 and Cx26 in hepatocytes, but not in liver epithelial cells, is increased by glucocorticoids, see for example, Kwiatkowski, A.P., et al., Carcinogenesis 15:1753-1758(1994), Ren, P., et al., Carcinogenesis 15:1807-1814(1994), and Ren, P. and Ruch, R.J., Carcinogenesis 17:2119-2124(1996). Estrogen increases Cx43 transcription (Yu, W. et al., Proc. R. Soc. Lond [Biol] 255:125-132(1994)). cAMP, forskolin, and glucagon increase Cx32 and Cx43 transcription in certain cells (see for example, Saez, J.C., et al., Proc. Natl. Acad. Sci USA 83:2473-2477(1986), Traub, O., et al., Eur. J. Cell Biol. 43:48-54(1987), Mehta, P.P., et al., Mol. Biol. Cell 3:839-850(1992), Burghardt, R.C., et al., J. Membr. Biol. 148:243-253(1995), and Matesic, D.F., et al, Neuroendocrin. 64:286-297(1996)). Therefore, the assay may be set up whereby formation of the gap junction is dependent upon expression of a connexin in the receiver cell and/or the sender cell. In one such assay, formation of a functional gap junction is dependent upon expression of Cx32 in the sender hepatocyte cell, the expression of which is increased by glucocorticoids. Alternatively, the assay may be set up to alter post-transcriptional modulation of a connexin of the sender and/or receiver cell, such as phosphorylation of the connexin, which in turn affects its stability and/or ability to form a functional gap junction. Degradation of Cx32-containing gap junctions can be mediated by the calcium-activated proteases, µ-calpain and m-calpain. and Cx50 is processed in the occular lens by calpain.

In yet another embodiment of the invention, the assay may be set up to determine the ability of a test compound for tumorigenic properties or anti-tumorigenic properties. Many oncogene proteins, such as ras, neu and src, block GJIC. Therefore, an assay may be set up to determine the ability of the test compound to alter gap junction through its ability to express or suppress an oncogene in either the sender or receiver cell.

In yet another embodiment of the invention, the assay conditions may be altered to determine if a putative connexin is capable of forming a functional gap junction with a known connexin. Thus, one aspect of the invention is a method to identify and characterize putative connexins, and to identify functional connexons with such putative connexins. In one aspect, a putative connexin is expressed in either the receiver or sender cell, and the other cell, either a sender or receiver cell respectively, expresses a known connexin. Upon stimulation with a known test compound, or under condition suitable to generate the endogenous messenger response, if a the receiver cell expresses the reporter response, then the putative connexin is identified as capable of forming a gap junction, and is thus, the putative connexin is identified as a functional connexin.

In yet another embodiment of the invention, one or more amino acids of a known connexin may be altered to determine the impact of such a change on the ability to form a functional connexon. The alteration may involve a single amino acid substitution for an amino acid of a similar size, polarity, hydrophobicity, or may involve a deletion or addition of one or more amino acids. If the endogenous messenger response is detected by virtue of inducing the reporter response, then the alteration of that amino acid, or alteration of that site, had little or no effect on the ability of the connexin to form a functional connexon. If the reporter response is not detected under conditions that activate the reporter response in the non-altered connexin, then the altered amino acid had a significant role in the ability to form a functional connexon. Thus, this embodiment of the invention permits identification of specific amino acids involved in formation of a functional connexon.

The sender cell may contain a membrane receptor for which a suitable agonist is known. Various receptors can be used including receptors which are endogenous to the sender cell. The only requirement which governs the choice of the sender cell receptor is that it not be present on the receiver cell. Generally, a cDNA molecule encoding a cell-surface receptor is introduced into the sender cell by transfection. Either transient transfection or stable expression can be used. It is preferred that the transfected receptor be stably expressed in the sender cell , which requires the use of a selectable marker and isolation of cells which have integrated the receptor-encoding DNA into their genome.

An 'exogenous stimulus' refers to any exogenous change in the assay reaction, and includes addition of molecules such as naturally-occurring ligands, agonists, antagonists, inverse agonists and the like of receptors, or other cell-permeable compounds. Cell permeable compounds would be appropriate if the receiver cell is incapable of responding to the cell-permeable compound. For example, if the receiver cell does not possess adenylate cyclase, a suitable exogenous stimulus might include a cell-permeable adenylate cyclase activator such as forskolin. Suitable exogenous stimuli also include chemical, physical, or any exogenous change in environmental condition of the assay reaction such as a change in temperature, that is capable of generating an endogenous messenger response in the first cell but not in the second cell. Preferred exogenous stimuli may bind to and activate one or more G-coupled protein receptors (GPCRs) or other receptors located on the cell surface, which in turn activate adenylate or guanylate cyclase resulting in increased levels of cAMP, or cGMP, respectively. Other pathways capable of initiating detectable endogenous messenger responses may be used to increase intracellar levels of calcium or inositol trisphosphate.

Various agonists may be used to activate the receptors on the sender cell. The choice of agonist is governed by its specificity for activating the sender cell without effects on the receiver cell. The agonist should be without inherent effects on gap junctional permeability, not have detrimental effects on cell viability, and be free of effect on the activity of the reporter system in the absence of the receptor.

Preferred endogenous messenger responses include any biological response generated by the first cell which is capable of passing through the gap junction and initiate a detectable response in the second cell. Initiation of a detectable response may be direct, such as increased levels of cyclic adenosine monophosphate (cAMP) in the first cell which in turn initiate, for example, transcription of a second reporter gene which is linked to a promoter-reporter construct containing a cAMP responsive element (CRE). If the endogenous messenger response is calcium, the detectable response may be aequorin activation in the receiver cell. Alternatively, if the endogenous messenger response is inositol trisphosphate, the detectable response may be a reporter gene which is linked to a promoter-reporter construct containing an SRE or TRE response element. Other responsive elements may be used in the present invention, such as a steroidal response element, for example, estrogen response element. Steroidal response elements includes both endogenous or naturally-occurring steroidal response elements and modified or mutant steroidal response elements.

Initiation of a detectable response may also be indirect, such as where increased levels of the messenger response in turn activate additional pathways, which at some point, produce a detectable response in the second cell. In one embodiment, the exogenous stimulus is capable of suppressing a constitutive protein only in the receiver cell and not in the sender cell, which could be achieved if the sender and receiver cells are of a different cell type, or the same if one or both are genetically altered to achieve this result. Initiation of a detectable response includes initiation of cell pathways such as apoptosis, where the endogenous messenger response increases levels of a protein which is a death signal which in turn initiates transcription of proteins in the apoptosis pathway. Where such a system is used, the detection method may be linked to cell viability or cell death.

A variety of reporter constructs are known in the art and may be used in the present method providing the reporter construct is appropriate for detecting the second messenger that is produced by activating the receptor on the sender cell. The reporter construct consists of a promoter sequence containing an element responsive to the second messenger-induced change. A promoter sequence is a DNA sequence that promotes transcription of a gene to produce mRNA and may be the attachment site for RNA polymerase (transcriptase). A promoter may comprise a TATA box, or a Pribnow box which is found in procaryotes. The promoter sequence is located in a position which permits it to regulate the transcription of a DNA sequence encoding an easily detected protein. Alternatively, the known reporters can be modified using molecular biological techniques to produce modified reporter systems with superiority in detecting the desired biochemical event. Examples of known promoter sequences include, but are not limited to, cAMP response element (CRE), serum response element (SRE), TPA response element (TRE). Examples of known, easily detectable, proteins are luciferase, β-galactosidase, chloramphenicol acetyl transferase, and aequorin.

Other examples of transcriptional control elements contemplated for use in the practice of the present invention include the vasoactive intestinal peptide gene promoter (cAMP responsive, Fink et al. (1988), Proc. Natl. Acad. Sci. 85:6662-6666)), the somatostatin gene promoter (cAMP responsive, Montminy et al. (1986), Proc. Natl. Acad. Sci. 83:6682-6686), the proenkephalin promoter (responsive to cAMP, nicotinic agonists, and phorbol esters, Comb et al. (1986), Nature 323:353-356), the phosphoenolpyruvate carboxykinase gene promoter (cAMP responsive; Short et al. (1986), J. Biol. Chem. 261:9721-9726), the NGFI-A gene promoter (responsive to NGF, cAMP, and serum; Changelian et al. (1989). Proc. Natl. Acad. Sci. 86:377-381), and the like.

**Table 3. Examples of selected receptor, reporter, and agonist combinations.**

| Receptor | Reporter | Agonist |
|---|---|---|
| D₁-dopamine | 6CRE-luciferase | Apomorphine |
| D₁-dopamine | 6CRE-β-Galactosidase | Apomorphine |
| α_{1A}-adrenergic | Mitochondrial aequorin | Phenylephrine |
| M₃-muscarinic acetylcholine | Mitochondrial aequorin | Carbamylcholine |
| β-adrenergic | 6-CRE luciferase | Isoproterenol |
| Vasoactive intestinal | 6-CRE luciferase | Vasoactive intestinal |
| polypeptide | | polypeptide |
| A_{2A}-adenosine | 6-CRE luciferase | CGS 21680 |
| 5HT₇- serotonin | 6-CRE luciferase | 8-hydroxy DPAT |
| P_{2y}-purinergic | Mitochondrial aequorin | ATP |

Detecting the reporter response' means analyzing the reaction mixture for the presence of the desired reporter molecule. The means used will depend upon the choice of reporter. If the reporter is expression of protein not normally expressed in the receiver cell, then a suitable detection means include methods for determining if the reporter protein is expressed. Expression of atypical or exogenous proteins is probably the most common second reporter, and any suitable protein, such as Luciferase, Green-fluorescent protein (GFP), and its many modified forms which fluorece at other wavelengths, or beta-galactosidase may be used and are readily detected by standard protocols. Alternatively, the second reporter might be inhibition of a protein normally expressed by the receiver cell. If the dectable response is initiation of apoptosis, detection of the reporter response might include use of viability stains, or other colorimetric dyes, or DNA laddering. In an apoptosis assay, only the receiver cell must become apoptotic and not the sender cell.

Additional reporter genes, in addition to those described above, include CAT (Alton and Vapnek (1979), Nature 282: 864-869); firefly luciferase (deWet et al. (1987), Mol. Cell. Biol. 7: 725-737); bacterial luciferase (Engebrecht and Silverman (1984); Proc. Natl. Acad. Sci. 1: 4154-4158; Baldwin et al. (1984), Biochemistry 23: 3663-3667); alkaline phosphatase (Toh et al. (1989), Eur. J. Biochem. 182: 231-238); and the like.

It is apparent that this assay will be sensitive both to agents which increase and those which decrease junctional permeability. The type of modulatory action will be evident from the output of the reporter system in the presence of the modulator relative to that observed in its absence. Responses which increase in the presence of the modulator substance indicate a substance which facilitates junctional permeability whereas responses which are of lower magnitude are indicative of a gap junction blocker.

In one embodiment of the present invention, the signal is increased intracellular cAMP levels caused by stimulation of a cell surface receptor, e.g. the dopamine D1-receptor, in the cell membrane of the "sender cells" with a suitable agonist. Activation of the receptor by the agonist produces a second messenger response. In the case of the D1 receptor, this response is an elevation of the intracellular concentration of cyclic adenosine monophosphate (cAMP). The detection of the second messenger signal is based on a transcription assay localized exclusively in the "receiver cells." The receiver cells contain a plasmid which has a promoter sequence sensitive to the biochemical consequences of the second messenger action. For example, D1 receptor-elicited cAMP increases can be detected by suitable promoter-reporter constructs containing the cAMP-responsive element (CRE) driving transcription of a DNA encoding a reporter enzyme such as firefly luciferase. The unique feature of the current invention is that the production of the second messenger and its detection occur in two different cells and will only be possible in the presence of a functional gap junction. When these conditions are satisfied, the intracellular concentration of cAMP increases in the receiver cells, a signaling cascade is set in motion which results in the activation of the CRE and transcription of the firefly luciferase enzyme, whose presence can be easily detected by a chemiluminescence assay.

Since the reporter protein will be produced only if there is GJIC between the sender and the receiver cells, this assay system can easily be utilized to identify agents which alter gap junctional permeability. Such compounds will be detected by alterations in the magnitude of the chemiluminescence signal in the receiver cells after addition of a receptor agonist, e.g. apomorphine activation of D1-dopamine receptors. Suitable substances which produce receptor-independent increases in the relevant second-messenger species are used to verify the integrity of the reporter system. In the example of the D1 receptor the diterpene forskolin, which is an adenylyl cyclase activator, can be used to elevate cAMP in both sender and receiver cells and increase levels of the reporter enzyme.

Two types of cells have been used in these experiments and each will be discussed separately. The first set of experiments concerns cell types known to express functional connexin channels endogenously, and therefore display efficient GJIC. The cell lines chosen to represent this class of cells are NRK (Normal Rat Kidney cells, described in Li, H., et al., J. Cell Biol., 134(4) : 1019-30, 1996) and CHO (Chinese Hamster Ovary cells).

Since many cells usually express multiple types of connexins and it is known that many different connexins can compensate for deficiencies of a defective connexin, native cells which express multiple types of connexins are less useful for studying specific members of the connexin family. Therefore, the examples also demonstrate the use of cells which have no endogenous detectable GJIC and are made to produce GJIC by transfection of plasmids carrying the appropriate connexin cDNA. The cell type exemplified herein for these experiments is the HeLa line (Graeber, S.H.M. and Hulser, D. Exp'l Cell Res., 243, 142-149, 1998).

All the experiments described below are performed according to the following protocol described below.

### Protocol

**Day 1:** Cells are plated at a density of approximately 1.5 million cells per 6 cm dish in the appropriate culture media.

**Day 2:** Sender and receiver cells are transfected separately with the various plasmids (for example, CRE-luciferase, D1-receptor, Cx32, Renilla Luciferase) according to the Optimem Plus protocol. Briefly, the appropriate amounts of DNA are pre-incubated for 15 minutes at room temperature with the Plus Reagent, then mixed with the diluted Lipofectamine reagent and incubated for another 15 minutes at room temperature. Then the transfection solution (1.5 ml per dish) is added dropwise to the culture dishes containing each 5 ml of Optimem media. The dishes are incubated at 37 degrees Celsius for 3 hours before 6.5 ml of Optimem media containing 20 % fetal bovine serum is added. The cells are then grown overnight at 37 degrees Celsius.

**Day 3:** The following steps are performed at the hours indicated: Time = 0 hours: The dishes are rinsed once with phosphate-buffered saline (PBS) and harvested in trypsin or Cell Dissolution Solution (Sigma). The cells are spun down (2 min at 500 rpm) and resuspended in 12-14 ml media. They are then dispensed into the wells of 12- or 24- well plates to a total volume of 1.2 ml or 0.5 ml, respectively. The proportion of sender: receiver cells varied from experiment to experiment (see below). The dishes are then placed back in the incubator to allow the cells to attach for two to three hours.
Time = 2 ½ hours: Treatment of cells with inhibitors (test compound in vehicle) or vehicle only, where applicable. Vehicle used in the present examples was DMSO and 0.1% ascorbic acid.
Time = 3 hours: Treatment of cells with stimulatory agents (apomorphine, forskolin, cAMP, each in vehicle) or vehicle only.
Time = 9 -11 hours: Cell harvest. The media is aspirated from the wells. In experiments using CHO or HeLa cells, the wells are rinsed once with PBS. This step is not done for the NRK cells, which detach more readily from the dish. The cells are then harvested in 200-250 µL (12-well plate) or 100 mL (24-well plate) using Passive Lysis Buffer from the Promega Dual Luciferase reporter kit (see below). The cell lysates are transferred to microfuge tubes and stored overnight at - 80 degrees Celsius.

**Day 4:** The samples are thawed on the bench and spun at 14,000 rpm for 2 minutes in a benchtop centrifuge. Twenty µL supernatant is transferred to white 96-well plates. Renilla and firefly luciferase activity is assayed using 50 µL of the reagents provided by the Promega Dual Luciferase kit.

### Calculation of results

Typcially, experiments are performed in triplicate or quadruplicate. The Firefly signal is normalized to the Renilla signal to account for differences in cell number and transfection efficiencies. Results are expressed as mean ± S.E.M.

### Materials

- Cell culture media for NRK and HeLa cells : Minimal Essential Medium with Earle's Balanced Salt Solution with 1 % glutamine solution (GIBCO-BRL), 10 % fetal bovine serum, 1 % sodium pyruvate and 1 % non-essential amino acids.
- Cell culture media for CHO cells : Ham F12 media, 10 % fetal bovine serum and 1 % penicillin-streptomycin solution (GIBCO-BRL)
- CHO-K1 cells, ATTC catalog # CCL-61
- HeLa cells, ATTC catalog # CCL-2
- NRK cells, ATTC catalog # CRL-6509
- pRL-SV40 (Promega E2231), pRL-CMV (Promega, E2261)
- Cell Dissociation Solution (Sigma, C5914)
- Lipofectamine Plus (GIBCO, 10964-013)
- Dual Luciferase Reporter Assay System (Promega, E1910)
- Apomorphine hydrochloride (Merck Chemical Co. 1161)
- 18-alpha-glycyrrhetinic acid (Sigma G8503)

Apomorphine is freshly diluted to a stock concentration of 10 mM in DMSO containing 0.1 % ascorbic acid for each experiment. Cyclic AMP is dissolved in water. All other compounds are dissolved in DMSO and 0.1 % ascorbic acid (vehicle). Apomorphine, cyclic AMP and forskolin are added as 1000x stocks. Compound additions represented a maximal 1.5 % of the well volume and identical volumes of vehicle controls are used.

### Example 1 Gap Junction Assay with Endogenous Gap Junction Connexins

### A. Experiments in cells with endogenous GJIC

NRK (Normal Rat Kidney) cells express mainly Cx43 and have efficient GJIC (Li, H., et al., J. Cell Biol., 134 (4) : 1019-30, 1996). Therefore, in these experiments no exogenous connexin DNA is transfected.

### A.1. Gap junction-mediated transfer of response from sender to receiver

Cells are transfected with 10 µg D1-receptor DNA and 500 ng pRLSV40 DNA (sender cells) or 10 µg CRE-luciferase DNA with 500 ng pRLSV40 DNA (receiver cells) per 6 cm dish. Cells are seeded in the wells of a 24-well plate in sender: receiver ratios of 5:1. Cells are treated with buffer or apomorphine for 6 hours before harvesting. The results are provided in Table 4, below. Values represent the mean ± SEM of 3 determinations.

**Table 4**

| Agonist | Receiver Cell Response |
|---|---|
| Buffer | 100 ± 4.9% |
| Apomorphine | 324 ± 14.4% |

### A.2. NRK cells do not express the endogenous D1-receptor

These experiments are performed to investigate whether the NRK cells express the D1-receptor endogenously. If NRK cells express D1 receptor endogenously, the receiver cells would be able to generate cAMP and produce a chemiluminescent signal independently of GJIC and the endogenous messenger generated by the sender cell. So only receiver cells are used for this experiment.

Cells are transfected with 5 µg CRE-luciferase DNA and 500 ng pRL-SV40 DNA per 6 cm dish. Each well of a 24-well plate received 250 µL cell suspension and 250 µL media, or approx. 25 000 cells. Cells are stimulated with 10µM Forskolin or apomorphine during 6 ½ hours before harvesting. Luciferase responses are 100.0 % ± 19.9 (control), 124.8 % ± 6.8 (apomorphine) and 876.6 % ± 64.1 (forskolin).

These results show that in NRK cells apomorphine is not able to elicit a response, indicating the absence of endogenous D1-receptor. The receptor-independent stimulator of cAMP, forskolin, in contrast, caused a more than 8-fold increase in chemiluminescent signal, indicating that the transfection of CRE-luciferase had been successful, and that the intracellular machinery required for relaying the signal from cAMP to luciferase expression is intact.

### A.3. Extrusion and re-uptake of cAMP cannot account for the observed activation of CRE-luciferase

The purpose of this assay is to measure GJIC. Cyclic AMP is chosen specifically as the signal molecule because it is known that gap junctions are permeable to it. Another possibility cannot be overlooked, however : cAMP, generated in the sender cells after stimulation of D1-receptors by apomorphine, may be extruded into the extracellular milieu and be taken up by passive diffusion through the cell membranes of the receiver cells. This would constitute a "false positive" signal for the presence of GJIC.
The likelihood of this sequence of events is small since cAMP is unstable and does not permeate membranes readily. It has been determined that IV injection of glucagon in humans can bring the plasma levels of cAMP up to 200 nM (Komatsu, R., Tsushima, N, Matsuyama, T. Clinical Hemorheology and Microcirculation, 17, 271-277, 1997.) We tested the effect of 1µM cAMP, exogenously added to the mix of sender and receiver cells.

Sender cells are transfected with 10 µg D1-receptor DNA and 500 ng pRL-SV40 per 6 cm dish; receiver cells with 5 µg CRE-luciferase and 500 ng pRL-SV40 per dish. Sender and receiver cells are mixed in a 1:1 ratio and seeded in 12-well dishes (1 ml or 100 000 cells/well). Cells are stimulated with 10µM forskolin or 1µM cAMP for 6 hours. Luciferase responses are 100.0 ± 1.9% (control), 669.3 ± 56.8% (forskolin) and 87.5 ± 5.8% (cAMP).

These results indicate that excess of exogenous cAMP does not activate the transcription of firefly luciferase in the receiver cells. Therefore, transmission of the cAMP signal from the sender to the receiver cells occurs via GJIC.

### A.4. Apomorphine induces a chemiluminescent signal in the receiver cells which can be specifically inhibited by a known inhibitor of GJIC

NRK sender cells are transfected with 5 µg D1-receptor DNA and 500 ng pRL-SV40 per 6-cm dish and NRK receiver cells with 5 µg CRE-luciferase DNA and 500 ng pRL-SV40. They are mixed in a 1:1 ratio and 500 µL (approx. 50,000 cells) are plated into 24-well plates. Cells are left to settle for 2 ½ hours at 37 degrees Celsius. Then three different concentrations of the inhibitor of GJIC, 18-alpha-glycyrrhetinic acid (18-AGA) (Davidson, J.S., Baumgarten, I.M., Harley, E.H., Biochem. and Biophys. Res. Comm., 134 (1), 29-36, 1986) are added. The inhibitor is added in a maximal volume of 1.5 % of total volume in DMSO. Controls received an identical volume of DMSO. Thirty minutes later, forskolin and apomorphine (10µM each) are added as 1000 x stock and left in contact for 6 ½ hours. The results are provided in Table 5, below.

**Table 5. Results**

| Agonist | Vehicle | 50 µM AGA | 100 µM AGA | 150 µM AGA |
|---|---|---|---|---|
| Control | 100.0 ± 19.4% | 100.0 ± 21.3% | 100.0 ± 5.2% | 100.0 ± 8.4% |
| Apomorphine | 297.5 ± 21.7% | 156.0 ± 8.9% | 137.6 ±9.3% | 109.7 ± 8.0% |
| Forskolin | 581.3 ± 47.2% | 732.6 ± 52.2% | 669.9 ± 18.3% | 685.8 ± 32.0% |

These results show that apomorphine is able to cause a three-fold increase over background of the chemiluminescent signal. Since only the receiver cells have the CRE-luciferase plasmid, this indicates the presence of GJIC. A known inhibitor of GJIC, 18-alpha-glycyrrhetinic acid, is able to decrease this effect in a concentration-dependent manner. This effect of AGA is not due to a nonspecific action on the cellular machinery responding to cAMP, since the response to forskolin, which is independent of GJIC, is unaffected.

### B. Experiments in cells without endogenous GJIC

HeLa cells are derived from a human malignancy and lack GJIC (Graeber, S.H.M., and Hulser, D.F., Expl Cell Res., 243, 142-149, 1998 and see experiments below). Using transient transfection, we confirmed the experiments described by George et al., in Biochem and Biophys Res Comm, 247 (3) : 785-789, 1998, which demonstrate that, following introduction of Cx32-encoding DNA, HeLa cells can be made to display GJIC. The following example further demonstrates that the present assay is able to detect the Cx32-mediated GJIC.
I. A nucleotide sequence containing six repeating CRE elements, TGACGTCA, SEQ ID NO. 1 is subcloned into pGL3 vector from Promega ( GenBank Accession Number U47295) at Sac I (first six bases, GAGCTC) and Blg II sites (last six bases, AGATCT). SEQ ID NO. 1 II. Human D1 receptor (obtained from Duke University, the sequence is also disclosed in Dearry, A., et al., Nature 347(6288), 72-76 (1990), Accession No. X55760) is subcloned into pcDNA3.1zeo (Invitrogen, V860-20) at Bam HI site. The nucleotides coding for the receptor are in capital letters with vector sequence in lower case.

### B.1. HeLa cells do not have GJIC unless transfected with Cx32 DNA

Six cm dishes of HeLa sender cells are transfected with 500 ng pRL-SV40, 5 µg D1-receptor DNA (sequence provided above) with or without 4.8 µg Cx32 DNA (Paul, D.L., J. Cell Biol. 103,123-134,1986). Receiver cells are transfected in the same way, but 5 µg CRE-luciferase is substituted for the D1-receptor DNA.

The transfected cells are harvested, resuspended in 14 ml media and plated (1 ml sender cells + 0.2 ml receiver cells) in the wells of 12-well plates. The cells are allowed to attach for 3 hours and are then stimulated with 10 µM apomorphine, 10 µM forskolin or vehicle control. The results are provided in Table 6, below. The results represent the mean ± SEM of duplicates from a single experiment.

**Table 6**

| | Cells not transfected with Cx32 * | Cells transfected with Cx32* |
|---|---|---|
| Control | 100.0 ± 6.6% | 100.0 ± 14.5% |
| | | |
| Apomorphine | 109.5 ± 1.1% | 217.5 ± 3.0% |
| Forskolin | 272.5 ± 40.9% | 254.6 ± 113.3% |

| | | |
|---|---|---|
| *Results shown are expressed as a percentage of control. | | |

These experiments show that apomorphine is only able to elicit a response in the receiver cells if Cx32 is co-transfected. This demonstrates that transfection of specific connexin cDNA can induce GJIC that can be measured by our assay. The absence of a response in the cells not transfected with Cx32 also indicates that the receiver cells do not possess the endogenous D1-receptor. This is confirmed by an experiment in which apomorphine is added to wells containing only receiver cells. As predicted, there is no elicited response. Luciferase activity values are 100.0 ± 6.5 % (control, n = 2) and 75.9 ± 3.0% (apomorphine, n = 2).

### B.2. The efficiency of GJIC can be modulated by the amount of Cx32 DNA transfected

Sender cells are transfected with 2.4 µg D1-receptor DNA, 500 ng pRL-CMV DNA and 2.4 µg (single dose) or 4.8 µg Cx32 DNA (double dose). Receiver cells are transfected with 2.4 µg Cx32 DNA, 500 ng pRL-CMV DNA and 2.4 µg CRE-luciferase.

Cells are harvested and resuspended in 14 ml media. They are plated in wells of 12-well dishes (1 ml donor cells, 0.2 ml receiver cells) and incubated for 30 minutes before addition of 10 µM apomorphine, 10 µM forskolin or control. The results are provided in Table 9, below. The data shown represent the mean ± SEM of duplicates.

**Table 9.**

| Agonist | Single dose Cx32 | Double dose Cx32 |
|---|---|---|
| Control | 100.0 ± 0.4% | 100.0 ± 12.3% |
| Apomorphine | 121.1 ± 7.2% | 176.5 ± 8.1% |
| Forskolin | 139.0 ± 14.3% | 195.1 ± 18.2% |

These data show that the efficiency of GJIC can be increased by using larger amounts of Cx32 DNA for transfection.

### C. Expression of exogenous connexin DNA in cells with GJIC

CHO cells (Chinese Hamster Ovary) cells are used in these experiments. CHO sender cells are transfected with 3.6 µg D1-receptor DNA, 500 ng pRL-SV40 and with or without 3.6 µg Cx32 DNA, per 6-cm dish. The CHO receiver cells are transfected with 3.6 µg CRE-luciferase DNA, 500 ng pRL-SV40 and with or without 3.5 µg Cx32 DNA.

Cells are resuspended in 14 ml media and plated (1 ml sender cells, 0.2 ml receiver cells) in wells of 12-well dishes. After a 3 hour incubation at 37 degrees Celsius, the cells are treated with 10 µM apomorphine, 10 µM forskolin or vehicle for 8 hours before harvesting. The results are provided in Table 8, below. The data shown below are the mean ± SEM of duplicate determinations.

**Table 8**

| Agonist | No exogenous Cx32 | With exogenous Cx32 |
|---|---|---|
| Control | 100.0 ± 13.2% | 100.0 ± 4.3% |
| Apomorphine | 222.6 ± 22.6% | 302.2 ± 2.2% |
| Forskolin | 701.9 ± 0.0% | 356.5 ± 21.8% |

These data indicate that expression of exogenous Cx32 against the background of endogenous GJIC can increase the cellular communication, probably via the formation of additional gap junction channels.

### D. Example of using different cell types for sender and receiver and utilizing an endogenous receptor on the sender.

One embodiment of this invention is to utilize different cell types for the sender and receiver cells. Moreover, in this embodiment it is possible to use an endogenous receptor if it is uniquely present on the sender cell. Chinese hamster ovary (CHO-K1) cells possess an endogenous corticotropin releasing factor (CRF) receptor coupled to stimulation of adenylate cyclase. Transfection of CHO-K1 cells with Cx32 as described below is used to produce a sender cell population. CHO-K1 monolayers are transfected with 500 ng pRL-SV40 and with or without 3.6 µg Cx32 DNA, per 6-cm dish. C6-glioma receiver cells are transfected with 3.6 µg CRE-luciferase DNA, 500 ng pRL-SV40 and with or without 3.5 µg Cx32 DNA.

Cells are resuspended in 14 ml media and plated (1 ml sender cells, 0.2 ml receiver cells) in wells of 12-well dishes. After a 3 hour incubation at 37 degrees Celsius, the cells are treated with 100 nM CRF, 10 µM forskolin or vehicle for 8 hours before harvesting. Increases in reporter activity of Cx32-transfected cells stimulated with CRF indicate GJIC.

The above examples are intended to be illustrative of the invention, and are not intended to be used to limit the scope of the invention.

## Claims

1. A method to identify a test compound capable of altering gap junction function, comprising
(1) adding a test compound to an assay reaction wherein the assay reaction comprises a sender cell and a receiver cell, wherein
(a) the sender cell (1) expresses a first connexin on the cell surface which forms a first connexon and (2) is capable of generating an endogenous messenger response in response to an exogenous stimulus;
(b) the receiver cell (1) expresses a second connexin on the cell surface which forms a second connexon, (2) is not capable of generating an endogenous messenger response to an exogenous stimulus, and (3) is capable of generating a reporter response in response to the endogenous messenger response generated by the sender cell;
(c) the first connexon and the second connexon form a gap junction;
(2) adding an exogenous stimulus to the assay reaction; and
(3) detecting the reporter response;
wherein the reporter response is transcription of a second reporter gene linked to a promoter-reporter construct containing a cAMP responsive element (CRE), or a reporter gene linked to a promoter-reporter construct containing an SRE or TRE response element.

2. The method according to claim 1, wherein the first connexin is a native connexin in the sender cell.

3. The method according to claim 2, wherein the native connexin is a mammalian connexin.

4. The method according to claim 3, wherein the mammalian connexin is selected from the group consisting of a human connexin, primate connexin, murine connexin, or rattus connexin.

5. The method according to claim 1, wherein the second connexin is a native connexin in the receiver cell.

6. The method according to claim 5, wherein the native connexin is a mammalian connexin.

7. The method according to claim 6, wherein the mammalian connexin is selected from the group consisting of a human connexin, primate connexin, murine connexin, or rattus connexin.

8. The method according to claim 1, wherein the first connexin or the second connexin is selected from the group consisting of a native mammalian connexin of cx26, cx30, cx30.3, cx31, cx31.1, cx32, cx33, cx36, cx37, cx40, cx43, cx45, cx 46, cx50, cx57, CXN-311, or variant thereof comprising one to ten amino acid differences than a native connexin.

9. The method according to claim 8, wherein the first connexin or the second connexin is selected from the group consisting of a native connexin of cx26, cx30, cx30.3, cx31, cx31.1, cx32, cx33, cx36, cx37, cx40, cx43, cx45, cx 46, cx50, cx57, CXN-311, or variant thereof comprising one to seven amino acid differences than a native connexin.

10. The method according to claim 9, wherein the first connexin or the second connexin is selected from the group consisting of a native connexin of cx26, cx30, cx30.3, cox31, cox31.1, cx32, cx33, cx36, cx37, cx40, cx43, cx45, cx 46, cx50, cx57, CXN-311, or variant thereof comprising one to four amino acid differences than a native connexin.

11. The method according to claim 10, wherein the first connexin or the second connexin is selected from the group consisting of a native connexin of cx26, cx30, cx30.3, cx31, cx31.1, cx32, cx33, cx36, cx37, cx40, cx43, cx45, cx 46, cx50, cx57, CXN-311, or variant thereof comprising one to three amino acid differences than a native connexin.

12. The method according to claim 11, wherein the first connexin or the second connexin is selected from the group consisting of a native connexin of cx26, cx30, cx30.3, cox31, cx31.1, cx32, cx33, cx36, cx37, cx40, cx43, cx45, cx 46, cx50, cx57, CXN-311, or variant thereof comprising one or two amino acid differences than a native connexin.

13. The method according to claim 12, wherein the first connexin or the second connexin is selected from the group consisting of a native connexin of cx26, cx30, cx30.3, cx31, cx31.1, cx32, cx33, cx36, cx37, cx40, cx43, cx45, cx 46, cx50, cx57, CXN-311, or variant thereof comprising one or two amino acid differences than a native connexin.

14. The method according to claim 1, wherein the first connexin is a non-native connexin in the sender cell.

15. The method according to claim 14, wherein the non-native connexin is a mammalian connexin.

16. The method according to claim 15, wherein the mammalian connexin is selected from the group consisting of a human connexin, primate connexin, murine connexin, or rattus connexin.

17. The method according to claim 16, wherein the second connexin is a non-native connexin in the receiver cell.

18. The method according to claim 17, wherein the non-native connexin is a mammalian connexin.

19. The method according to claim 18, wherein the mammalian connexin is selected from the group consisting of a human connexin, primate connexin, murine connexin, or rattus connexin.

20. The method according to claim 1, wherein the first connexin or the second connexin is selected from the group consisting of a non-native mammalian connexin of cx26, cx30, cx30.3, cox31, cx31.1, cx32, cx33, cx36, cx37, cx40, cx43, cx45, cx 46, cx50, cx57, CXN-311, or variant thereof comprising one to ten amino acid differences than a native connexin.

21. The method according to claim 20, wherein the first connexin or the second connexin is selected from the group consisting of a non-native connexin of cx26, cx30, cx30.3, cox31, cx31.1, cx32, cx33, cx36, cx37, cx40, cx43, cx45, cx 46, cx50, cx57, CXN-311, or variant thereof comprising one to seven amino acid differences than a native connexin.

22. The method according to claim 21, wherein the first connexin or the second connexin is selected from the group consisting of a non-native connexin of cx26, cx30, cx30.3, cx31, cx31.1, cx32, cx33, cx36, cx37, cx40, cx43, cx45, cx 46, cx50, cx57, CXN-311, or variant thereof comprising one to four amino acid differences than a native connexin.

23. The method according to claim 22, wherein the first connexin or the second connexin is selected from the group consisting of a non-native connexin of cx26, cx30, cx30.3, cox31, cx31.1, cx32, cx33, cx36, cx37, cx40, cx43, cx45, cx 46, cx50, cx57, CXN-311, or variant thereof comprising one to three amino acid differences than a native connexin.

24. The method according to claim 23, wherein the first connexin or the second connexin is selected from the group consisting of a non-native connexin of cx26, cx30, cx30.3, cox31, cx31.1, cx32, cx33, cx36, cx37, cx40, cx43, cx45, cx 46, cx50, cx57, CXN-311, or variant thereof comprising one or two amino acid differences than a native connexin.

25. The method according to claim 24, wherein the first connexin or the second connexin is selected from the group consisting of a non-native connexin of cx26, cx30, cx30.3, cox31, cx31.1, cx32, cx33, cx36, cx37, cx40, cx43, cx45, cx 46, cx50, cx57, CXN-311, or variant thereof comprising one or two amino acid differences than a native connexin.

26. The method according to claim 1, wherein the first connexin or the second connexin is a non-naturally-occurring connexin.

27. The method according to claim 26, wherein the non-naturally-occurring connexin is a chimera connexin where one or more amino acids in one or more domains of a native connexin is substituted with amino acids of a corresponding domain in a different native connexin.

28. The method according to claim 26, wherein the non-naturally-occurring connexin is a chimera connexin where one or more amino acids in one or more domains of a native connexin is substituted with an amino acid of a similar charge, polarity, or size, or is deleted.

29. The method according to claim 26, wherein the non-naturally-occurring connexin is a chimera connexin where a phosphorylated-region of a carboxy tail of phosphorylated connexin is substituted with, or added to, a native connexin which is not normally phosphorylated.

30. The method according to claim 1, wherein the endogenous messenger response is increased level of cyclic adenosine monophosphate (cAMP), calcium, or inositol trisphosphate.

31. The method according to claim 1, wherein the reporter response is detected by measuring the level of expression an exogenous protein.

32. The method according to claim 31, wherein the exogenous protein is selected from the group consisting of firefly luciferase, bacterial luciferase, alkaline phosphatase, Green-fluorescent protein (GFP), and a modified form of GFP which fluoresces at a different wavelength than GFP, beta-galactosidase, and CAT.

33. A method to identify a test compound capable of altering gap junction function, comprising
(1) adding an exogenous stimulus to an assay reaction, wherein the assay reaction comprises a sender cell and a receiver cell, wherein
(a) the sender cell (1) expresses a first connexin on the cell surface which forms a first connexon and (2) is capable of generating an endogenous messenger response in response to an exogenous stimulus;
(b) the receiver cell (1) expresses a second connexin on the cell surface which forms a second connexon, (2) is not capable of generating an endogenous messenger response to an exogenous stimulus, and (3) is capable of generating a reporter response in response to the endogenous messenger response generated by the sender cell;
(c) the first connexon and the second connexon form a gap junction;
(2) adding a test compound to the assay reaction; and
(3) detecting the reporter response;
wherein the reporter response is transcription of a second reporter gene linked to a promoter-reporter construct containing a cAMP responsive element (CRE), or a reporter gene linked to a promoter-reporter construct containing an SRE or TRE response element.

34. The method according to claim 33, wherein the first connexin is a native connexin in the sender cell.

35. The method according to claim 34, wherein the native connexin is a mammalian connexin.

36. The method according to claim 35, wherein the mammalian connexin is selected from the group consisting of a human connexin, primate connexin, murine connexin, or rattus connexin.

37. The method according to claim 33, wherein the second connexin is a native connexin in the receiver cell.

38. The method according to claim 37, wherein the native connexin is a mammalian connexin.

39. The method according to claim 38 wherein the mammalian connexin is selected from the group consisting of a human connexin, primate connexin, murine connexin, or rattus connexin.

40. The method according to claim 33, wherein the first connexin or the second connexin is selected from the group consisting of a native mammalian connexin of cx26, cx30, cx30.3, cox31, cx31.1, cx32, cx33, cx36, cx37, cx40, cx43, cx45, cx 46, cx50, cx57, CXN-311, or variant thereof comprising one to ten amino acid differences than a native connexin.

41. The method according to claim 40, wherein the first connexin or the second connexin is selected from the group consisting of a native connexin of cx26, cx30, cx30.3, cox31, cox31.1, cx32, cx33, cx36, cx37, cx40, cx43, cx45, cx 46, cx50, cx57, CXN-311, or variant thereof comprising one to seven amino acid differences than a native connexin.

42. The method according to claim 41, wherein the first connexin or the second connexin is selected from the group consisting of a native connexin of cx26, cx30, cx30.3, cox31, cx31.1, cx32, cx33, cx36, cx37, cx40, cx43, cx45, cx 46, cx50, cx57, CXN-311, or variant thereof comprising one to four amino acid differences than a native connexin.

43. The method according to claim 42, wherein the first connexin or the second connexin is selected from the group consisting of a native connexin of cx26, cx30, cx30.3, cx31, cx31.1, cx32, cx33, cx36, cx37, cx40, cx43, cx45, cx 46, cx50, cx57, CXN-311, or variant thereof comprising one to three amino acid differences than a native connexin.

44. The method according to claim 43, wherein the first connexin or the second connexin is selected from the group consisting of a native connexin of cx26, cx30, cx30.3, cx31, cx31.1, cx32, cx33, cx36, cx37, cx40, cx43, cx45, cx 46, cx50, cx57, CXN-311, or variant thereof comprising one or two amino acid differences than a native connexin.

45. The method according to claim 44, wherein the first connexin or the second connexin is selected from the group consisting of a native connexin of cx26, cx30, cx30.3, cx31, cx31.1, cx32, cx33, cx36, cx37, cx40, cx43, cx45, cx 46, cx50, cx57, CXN-311, or variant thereof comprising one or two amino acid differences than a native connexin.

46. The method according to claim 33, wherein the first connexin is a non-native connexin in the sender cell.

47. The method according to claim 46, wherein the non-native connexin is a mammalian connexin.

48. The method according to claim 47, wherein the mammalian connexin is selected from the group consisting of a human connexin, primate connexin, murine connexin, or rattus connexin.

49. The method according to claim 48, wherein the second connexin is a non-native connexin in the receiver cell.

50. The method according to claim 49, wherein the non-native connexin is a mammalian connexin.

51. The method according to claim 50, wherein the mammalian connexin is selected from the group consisting of a human connexin, primate connexin, murine connexin, or rattus connexin.

52. The method according to claim 33, wherein the first connexin or the second connexin is selected from the group consisting of a non-native mammalian connexin of cx26, cx30, cx30.3, cx31, cx31.1, cx32, cx33, cx36, cx37, cx40, cx43, cx45, cx 46, cx50, cx57, CXN-311, or variant thereof comprising one to ten amino acid differences than a native connexin.

53. The method according to claim 52, wherein the first connexin or the second connexin is selected from the group consisting of a non-native connexin of cx26, cx30, cx30.3, cx31, cx31.1, cx32, cx33, cx36, cx37, cx40, cx43, cx45, cx 46, cx50, cx57, CXN-311, or variant thereof comprising one to seven amino acid differences than a native connexin.

54. The method according to claim 53, wherein the first connexin or the second connexin is selected from the group consisting of a non-native connexin of cx26, cx30, cx30.3, cx31, cx31.1, cx32, cx33, cx36, cx37, cx40, cx43, cx45, cx 46, cx50, cx57, CXN-311, or variant thereof comprising one to four amino acid differences than a native connexin.

55. The method according to claim 54, wherein the first connexin or the second connexin is selected from the group consisting of a non-native connexin of cx26, cx30, cx30.3, cx31, cx31.1, cx32, cx33, cx36, cx37; cx40, cx43, cx45, cx 46, cx50, cx57, CXN-311, or variant thereof comprising one to three amino acid differences than a native connexin.

56. The method according to claim 55, wherein the first connexin or the second connexin is selected from the group consisting of a non-native connexin of cx26, cx30, cx30.3, cx31, cx31.1, cx32, cx33, cx36, cx37, cx40, cx43, cx45, cx 46, cx50, cx57, CXN-311, or variant thereof comprising one or two amino acid differences than a native connexin.

57. The method according to claim 56, wherein the first connexin or the second connexin is selected from the group consisting of a non-native connexin of cx26, cx30, cx30.3, cx31, cx31.1, cx32, cx33, cx36, cx37, cx40, cx43, cx45, cx 46, cx50, cx57, CXN-311, or variant thereof comprising one or two amino acid differences than a native connexin.

58. The method according to claim 33, wherein the first connexin or the second connexin is a non-naturally-occurring connexin.

59. The method according to claim 58, wherein the non-naturally-occurring connexin is a chimera connexin where one or more amino acids in one or more domains of a native connexin is substituted with amino acids of a corresponding domain in a different native connexin.

60. The method according to claim 59, wherein the non-naturally-occurring connexin is a chimera connexin where one or more amino acids in one or more domains of a native connexin is substituted with an amino acid of a similar charge, polarity, or size, or is deleted.

61. The method according to claim 59, wherein the non-naturally-occurring connexin is a chimera connexin where a phosphorylated-region of a carboxy tail of phosphorylated connexin is substituted with, or added to, a native connexin which is not normally phosphorylated.

62. The method according to claim 33, wherein the endogenous messenger response is increased level of cyclic adenosine monophosphate (cAMP), calcium, or inositol trisphosphate.

63. The method according to claim 33, wherein the reporter response is detected by measuring the level of expression an exogenous protein.

64. The method according to claim 63, wherein the exogenous protein is selected from the group consisting of firefly luciferase, bacterial luciferase, alkaline phosphatase, Green-fluorescent protein (GFP), and a modified form of GFP which fluoresces at a different wavelength than GFP, beta-galactosidase, and CAT.

## Patentansprüche

1. Verfahren zur Identifizierung einer zur Änderung der "Gap Junction"-Funktion fähigen Testverbindung, bei dem man
(1) eine Testverbindung zu einer Assay-Reaktion gibt, wobei die Assay-Reaktion eine Senderzelle und eine Empfängerzelle umfasst, wobei
(a) die Senderzelle (1) ein erstes Connexin auf der Zelloberfläche unter Bildung eines ersten Connexons exprimiert und (2) in der Lage ist, eine endogene Botenantwort als Antwort auf einen exogenen Reiz zu erzeugen;
(b) die Empfängerzelle (1) ein zweites Connexin auf der Zelloberfläche unter Bildung eines zweiten Connexons exprimiert, (2) nicht in der Lage ist, eine endogene Botenantwort als Antwort auf einen exogenen Reiz zu erzeugen und (3) in der Lage ist, eine Reporterantwort als Antwort auf die durch die Senderzelle erzeugte endogene Botenantwort zu erzeugen;
(c) das erste Connexon und das zweite Connexon eine Gap Junction bilden;
(2) einen exogenen Reiz zu der Assay-Reaktion gibt und
(3) die Reporterantwort nachweist,
wobei es sich bei der Reporterantwort um die Transkription eines mit einem ein auf cAMP reagierendes Element (CRE) enthaltenden Promotor-Reporter-Konstrukt verknüpften zweiten Reportergens oder eines mit einem ein SRE- oder TRE-Antwort-Element enthaltenden Promotor-Reporter-Konstrukt verknüpften Reportergens handelt.

2. Verfahren nach Anspruch 1, wobei es sich bei dem ersten Connexin um ein natives Connexin in der Senderzelle handelt.

3. Verfahren nach Anspruch 2, wobei es sich bei dem nativen Connexin um ein Säuger-Connexin handelt.

4. Verfahren nach Anspruch 3, wobei das Säuger-Connexin aus der Gruppe menschliches Connexin, Primaten-Connexin, Mäuse-Connexin oder Ratten-Connexin ausgewählt ist.

5. Verfahren nach Anspruch 1, wobei es sich bei dem zweiten Connexin um ein natives Connexin in der Empfängerzelle handelt.

6. Verfahren nach Anspruch 5, wobei es sich bei dem nativen Connexin um ein Säuger-Connexin handelt.

7. Verfahren nach Anspruch 6, wobei das Säuger-Connexin aus der Gruppe menschliches Connexin, Primaten-Connexin, Mäuse-Connexin oder Ratten-Connexin ausgewählt ist.

8. Verfahren nach Anspruch 1, wobei das erste Connexin oder das zweite Connexin aus der Gruppe natives Säuger-Connexin von cx26, cx30, cx30.3, cx31, cx31.1, cx32, cx33, cx36, cx37, cx40, cx43, cx45, cx46, cx50, cx57, CXN-311 oder Variante davon mit einem bis zehn Aminosäureunterschieden zu einem nativen Connexin ausgewählt ist.

9. Verfahren nach Anspruch 8, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem bis sieben Aminosäureunterschieden zu einem nativen Connexin ausgewählt ist.

10. Verfahren nach Anspruch 9, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem bis vier Aminosäureunterschieden zu einem nativen Connexin ausgewählt ist.

11. Verfahren nach Anspruch 10, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem bis drei Aminosäureunterschieden zu einem nativen Connexin ausgewählt ist.

12. Verfahren nach Anspruch 11, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

13. Verfahren nach Anspruch 12, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

14. Verfahren nach Anspruch 1, wobei es sich bei dem ersten Connexin um ein nichtnatives Connexin in der Senderzelle handelt.

15. Verfahren nach Anspruch 14, wobei es sich bei dem nichtnativen Connexin um ein Säuger-Connexin handelt.

16. Verfahren nach Anspruch 15, wobei das Säuger-Connexin aus der Gruppe menschliches Connexin, Primaten-Connexin, Mäuse-Connexin oder Ratten-Connexin ausgewählt ist.

17. Verfahren nach Anspruch 16, wobei es sich bei dem zweiten Connexin um ein nichtnatives Connexin in der Empfängerzelle handelt.

18. Verfahren nach Anspruch 17, wobei es sich bei dem nichtnativen Connexin um ein Säuger-Connexin handelt.

19. Verfahren nach Anspruch 18, wobei das Säuger-Connexin aus der Gruppe menschliches Connexin, Primaten-Connexin, Mäuse-Connexin oder Ratten-Connexin ausgewählt ist.

20. Verfahren nach Anspruch 1, wobei das erste Connexin oder das zweite Connexin aus der Gruppe nichtnatives Säuger-Connexin von cx26, cx30, cx30.3, cm31, cx31.1, cx32, cx33, cx36, cx37, cx40, cx43, cx45, cx46, cx50, cx57, CXN-311 oder Variante davon mit einem bis zehn Aminosäureunterschieden zu einem nativen Connexin ausgewählt ist.

21. Verfahren nach Anspruch 20, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem bis sieben Aminosäureunterschieden zu einem nativen Connexin ausgewählt ist.

22. Verfahren nach Anspruch 21, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem bis vier Aminosäureunterschieden zu einem nativen Connexin ausgewählt ist.

23. Verfahren nach Anspruch 22, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem bis drei Aminosäureunterschieden zu einem nativen Connexin ausgewählt ist.

24. Verfahren nach Anspruch 23, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

25. Verfahren nach Anspruch 24, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

26. Verfahren nach Anspruch 1, wobei es sich bei dem ersten Connexin oder dem zweiten Connexin um ein nicht natürlich vorkommendes Connexin handelt.

27. Verfahren nach Anspruch 26, wobei es sich bei dem nicht natürlich vorkommenden Connexin um ein Chimären-Connexin handelt, bei dem eine oder mehrere Aminosäuren in einer oder mehreren Domänen eines nativen Connexins gegen Aminosäuren einer entsprechenden Domäne in einem anderen nativen Connexin ausgetauscht ist.

28. Verfahren nach Anspruch 26, wobei es sich bei dem nicht natürlich vorkommenden Connexin um ein Chimären-Connexin handelt, bei dem eine oder mehrere Aminosäuren in einer oder mehreren Domänen eines nativen Connexins gegen eine Aminosäure mit einer ähnlichen Ladung, Polarität oder Größe ausgetauscht oder deletiert ist.

29. Verfahren nach Anspruch 26, wobei es sich bei dem nicht natürlich vorkommenden Connexin um ein Chimären-Connexin handelt, bei dem ein phosphorylierter Bereich eines Carboxyendes von phosphoryliertem Connexin gegen ein natives Connexin, das normalerweise nicht phosphoryliert ist, ausgetauscht oder an dieses angefügt ist.

30. Verfahren nach Anspruch 1, wobei es sich bei der endogenen Botenantwort um eine erhöhte Konzentration an cyclischem Adenosinmonophosphat (cAMP), Calcium oder Inositoltrisphosphat handelt.

31. Verfahren nach Anspruch 1, wobei die Reporterantwort durch Messen des Expressionsniveaus eines exogenen Proteins nachgewiesen wird.

32. Verfahren nach Anspruch 31, wobei das exogene Protein aus der Gruppe Firefly-Luciferase, Bakterien-Luciferase, alkalische Phosphatase, grünes Fluoreszenzprotein (GFP) sowie eine modifizierte Form von GFP, die eine andere Fluoreszenzwellenlänge als GFP aufweist, Beta-Galactosidase und CAT ausgewählt ist.

33. Verfahren zur Identifizierung einer zur Änderung der "Gap Junction"-Funktion fähigen Testverbindung, bei dem man
(1) einen exogenen Reiz zu einer Assay-Reaktion gibt, wobei die Assay-Reaktion eine Senderzelle und eine Empfängerzelle umfasst, wobei
(a) die Senderzelle (1) ein erstes Connexin auf der Zelloberfläche unter Bildung eines ersten Connexons exprimiert und (2) in der Lage ist, eine endogene Botenantwort als Antwort auf einen exogenen Reiz zu erzeugen;
(b) die Empfängerzelle (1) ein zweites Connexin auf der Zelloberfläche unter Bildung eines zweiten Connexons exprimiert, (2) nicht in der Lage ist, eine endogene Botenantwort als Antwort auf einen exogenen Reiz zu erzeugen und (3) in der Lage ist, eine Reporterantwort als Antwort auf die durch die Senderzelle erzeugte endogene Botenantwort zu erzeugen;
(c) das erste Connexon und das zweite Connexon eine Gap Junction bilden;
(2) eine Testverbindung zu der Assay-Reaktion gibt und
(3) die Reporterantwort nachweist,
wobei es sich bei der Reporterantwort um die Transkription eines mit einem ein auf cAMP reagierendes Element (CRE) enthaltenden Promotor-Reporter-Konstrukt verknüpften zweiten Reportergens oder eines mit einem ein SRE- oder TRE-Antwort-Element enthaltenden Promotor-Reporter-Konstrukt verknüpften Reportergens handelt.

34. Verfahren nach Anspruch 33, wobei es sich bei dem ersten Connexin um ein natives Connexin in der Senderzelle handelt.

35. Verfahren nach Anspruch 34, wobei es sich bei dem nativen Connexin um ein Säuger-Connexin handelt.

36. Verfahren nach Anspruch 35, wobei das Säuger-Connexin aus der Gruppe menschliches Connexin, Primaten-Connexin, Mäuse-Connexin oder Ratten-Connexin ausgewählt ist.

37. Verfahren nach Anspruch 33, wobei es sich bei dem zweiten Connexin um ein natives Connexin in der Empfängerzelle handelt.

38. Verfahren nach Anspruch 37, wobei es sich bei dem nativen Connexin um ein Säuger-Connexin handelt.

39. Verfahren nach Anspruch 38, wobei das Säuger-Connexin aus der Gruppe menschliches Connexin, Primaten-Connexin, Mäuse-Connexin oder Ratten-Connexin ausgewählt ist.

40. Verfahren nach Anspruch 33, wobei das erste Connexin oder das zweite Connexin aus der Gruppe natives Säuger-Connexin von cx26, cx30, cx30.3, cm31, cx31.1, cx32, cx33, cx36, cx37, cx40, cx43, cx45, cx46, cx50, cx57, CXN-311 oder Variante davon mit einem bis zehn Aminosäureunterschieden zu einem nativen Connexin ausgewählt ist.

41. Verfahren nach Anspruch 40, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem bis sieben Aminosäureunterschieden zu einem nativen Connexin ausgewählt ist.

42. Verfahren nach Anspruch 41, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem bis vier Aminosäureunterschieden zu einem nativen Connexin ausgewählt ist.

43. Verfahren nach Anspruch 42, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem bis drei Aminosäureunterschieden zu einem nativen Connexin ausgewählt ist.

44. Verfahren nach Anspruch 43, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

45. Verfahren nach Anspruch 44, wobei das erste Connexin oder das zweite Connexin aus der Gruppe natives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

46. Verfahren nach Anspruch 33, wobei es sich bei dem ersten Connexin um ein nichtnatives Connexin in der Senderzelle handelt.

47. Verfahren nach Anspruch 46, wobei es sich bei dem nichtnativen Connexin um ein Säuger-Connexin handelt.

48. Verfahren nach Anspruch 47, wobei das Säuger-Connexin aus der Gruppe menschliches Connexin, Primaten-Connexin, Mäuse-Connexin oder Ratten-Connexin ausgewählt ist.

49. Verfahren nach Anspruch 48, wobei es sich bei dem zweiten Connexin um ein nichtnatives Connexin in der Empfängerzelle handelt.

50. Verfahren nach Anspruch 49, wobei es sich bei dem nichtnativen Connexin um ein Säuger-Connexin handelt.

51. Verfahren nach Anspruch 50, wobei das Säuger-Connexin aus der Gruppe menschliches Connexin, Primaten-Connexin, Mäuse-Connexin oder Ratten-Connexin ausgewählt ist.

52. Verfahren nach Anspruch 33, wobei das erste Connexin oder das zweite Connexin aus der Gruppe nichtnatives Säuger-Connexin von cx26, cx30, cx30.3, cm31, cx31.1, cx32, cx33, cx36, cx37, cx40, cx43, cx45, cx46, cx50, cx57, CXN-311 oder Variante davon mit einem bis zehn Aminosäureunterschieden zu einem nativen Connexin ausgewählt ist.

53. Verfahren nach Anspruch 52, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem bis sieben Aminosäureunterschieden zu einem nativen Connexin ausgewählt ist.

54. Verfahren nach Anspruch 53, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem bis vier Aminosäureunterschieden zu einem nativen Connexin ausgewählt ist.

55. Verfahren nach Anspruch 54, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem bis drei Aminosäureunterschieden zu einem nativen Connexin ausgewählt ist.

56. Verfahren nach Anspruch 55, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

57. Verfahren nach Anspruch 56, wobei das erste Connexin oder das zweite Connexin aus der Gruppe nichtnatives Connexin von cx26, cx30, cx30.3, cx31, cx31.1, cx32, cx33, cx36, cx37, cx40, cx43, cx45, cx46, cx50, cx57, CXN-311 oder Variante davon mit einem oder zwei Aminosäureunterschieden zu einem nativen Connexin ausgewählt ist.

58. Verfahren nach Anspruch 33, wobei es sich bei dem ersten Connexin oder dem zweiten Connexin um ein nicht natürlich vorkommendes Connexin handelt.

59. Verfahren nach Anspruch 58, wobei es sich bei dem nicht natürlich vorkommenden Connexin um ein Chimären-Connexin handelt, bei dem eine oder mehrere Aminosäuren in einer oder mehreren Domänen eines nativen Connexins gegen Aminosäuren einer entsprechenden Domäne in einem anderen nativen Connexin ausgetauscht ist.

60. Verfahren nach Anspruch 59, wobei es sich bei dem nicht natürlich vorkommenden Connexin um ein Chimären-Connexin handelt, bei dem eine oder mehrere Aminosäuren in einer oder mehreren Domänen eines nativen Connexins gegen eine Aminosäure mit einer ähnlichen Ladung, Polarität oder Größe ausgetauscht oder deletiert ist.

61. Verfahren nach Anspruch 59, wobei es sich bei dem nicht natürlich vorkommenden Connexin um ein Chimären-Connexin handelt, bei dem ein phosphorylierter Bereich eines Carboxyendes von phosphoryliertem Connexin gegen ein natives Connexin, das normalerweise nicht phosphoryliert ist, ausgetauscht oder an dieses angefügt ist.

62. Verfahren nach Anspruch 33, wobei es sich bei der endogenen Botenantwort um eine erhöhte Konzentration an cyclischem Adenosinmonophosphat (cAMP), Calcium oder Inositoltrisphosphat handelt.

63. Verfahren nach Anspruch 33, wobei die Reporterantwort durch Messen des Expressionsniveaus eines exogenen Proteins nachgewiesen wird.

64. Verfahren nach Anspruch 63, wobei das exogene Protein aus der Gruppe Firefly-Luciferase, Bakterien-Luciferase, alkalische Phosphatase, grünes Fluoreszenzprotein (GFP) sowie eine modifizierte Form von GFP, die eine andere Fluoreszenzwellenlänge als GFP aufweist, Beta-Galactosidase und CAT ausgewählt ist.

## Revendications

1. Procédé pour identifier un composé à tester capable d'altérer la fonction de jonction communicante, comprenant les étapes consistant à :
(1) ajouter un composé à tester à une réaction d'essai, dans lequel la réaction d'essai comprend une cellule émettrice et une cellule receveuse, dans laquelle
(a) la cellule émettrice : (1) exprime une première connexine à la surface cellulaire, laquelle forme un premier connexon ; et (2) est capable de générer une réponse de messager endogène en réponse à un stimulus exogène ;
(b) la cellule receveuse : (1) exprime une deuxième connexine à la surface cellulaire, laquelle forme un deuxième connexon ; (2) n'est pas capable de générer une réponse de messager endogène en réponse à un stimulus exogène ; et (3) est capable de générer une réponse rapporteuse en réponse à la réponse de messager endogène générée par la cellule émettrice ;
(c) le premier connexon et le deuxième connexon forment une jonction communicante ;
(2) ajouter un stimulus exogène à la réaction d'essai ; et
(3) détecter la réponse rapporteuse ;
dans lequel la réponse rapporteuse est la transcription d'un deuxième gène rapporteur lié à un construit promoteur-rapporteur, contenant un élément répondant à l'AMPc (CRE), ou un gène rapporteur lié à un construit promoteur-rapporteur contenant un élément de réponse SRE ou TRE.

2. Procédé selon la revendication 1, dans lequel la première connexine est une connexine naturelle dans la cellule émettrice.

3. Procédé selon la revendication 2, dans lequel la connexine naturelle est une connexine de mammifère.

4. Procédé selon la revendication 3, dans lequel la connexine de mammifère est choisie dans le groupe constitué d'une connexine humaine, une connexine de primate, une connexine murine ou une connexine de rat.

5. Procédé selon la revendication 1, dans lequel la deuxième connexine est une connexine naturelle dans la cellule receveuse.

6. Procédé selon la revendication 5, dans lequel la connexine naturelle est une connexine de mammifère.

7. Procédé selon la revendication 6, dans lequel la connexine de mammifère est choisie dans le groupe constitué d'une connexine humaine, une connexine de primate, une connexine murine ou une connexine de rat.

8. Procédé selon la revendication 1, dans lequel la première connexine ou la deuxième connexine est choisie dans le groupe constitué d'une connexine naturelle de mammifère cx26, cx30, cx30.3, cx3l, cx31.1, cx32, cx33, cx36, cx37, cx40, cx43, cx45, cx46, cx50, cx57, CXN-311 ou un variant de celles-ci, comprenant d'une à dix différences d'acides aminés par rapport à une connexine naturelle.

9. Procédé selon la revendication 8, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à sept différences d'acides aminés par rapport à une connexine naturelle.

10. Procédé selon la revendication 9, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à quatre différences d'acides aminés par rapport à une connexine naturelle.

11. Procédé selon la revendication 10, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à trois différences d'acides aminés par rapport à une connexine naturelle.

12. Procédé selon la revendication 11, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

13. Procédé selon la revendication 12, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

14. Procédé selon la revendication 1, dans lequel la première connexine est une connexine non naturelle dans la cellule émettrice.

15. Procédé selon la revendication 14, dans lequel la connexine non naturelle est une connexine de mammifère.

16. Procédé selon la revendication 15, dans lequel la connexine de mammifère est choisie dans le groupe constitué d'une connexine humaine, une connexine de primate, une connexine murine ou une connexine de rat.

17. Procédé selon la revendication 16, dans lequel la deuxième connexine est une connexine non naturelle dans la cellule receveuse.

18. Procédé selon la revendication 17, dans lequel la connexine non naturelle est une connexine de mammifère.

19. Procédé selon la revendication 18, dans lequel la connexine de mammifère est choisie dans le groupe constitué d'une connexine humaine, une connexine de primate, une connexine murine ou une connexine de rat.

20. Procédé selon la revendication 1, dans lequel la première connexine ou la deuxième connexine est choisie dans le groupe constitué d'une connexine non naturelle de mammifère cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à dix différences d'acides aminés par rapport à une connexine naturelle.

21. Procédé selon la revendication 20, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à sept différences d'acides aminés par rapport à une connexine naturelle.

22. Procédé selon la revendication 21, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à quatre différences d'acides aminés par rapport à une connexine naturelle.

23. Procédé selon la revendication 22, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à trois différences d'acides aminés par rapport à une connexine naturelle.

24. Procédé selon la revendication 23, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

25. Procédé selon la revendication 24, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

26. Procédé selon la revendication 1, dans lequel la première connexine ou la deuxième connexine est une connexine n'existant pas naturellement.

27. Procédé selon la revendication 26, dans lequel la connexine n'existant pas naturellement est une connexine chimère, dans laquelle un ou plusieurs acide(s) aminé(s) dans un ou plusieurs domaine(s) d'une connexine naturelle est (sont) substitué(s) par des acides aminés d'un domaine correspondant dans une connexine naturelle différente.

28. Procédé selon la revendication 26, dans lequel la connexine n'existant pas naturellement est une connexine chimère, dans laquelle un ou plusieurs acide(s) aminé(s) dans un ou plusieurs domaine(s) d'une connexine naturelle est (sont) substitué(s) par un acide aminé ayant une charge, une polarité ou une taille semblable ou bien est (sont) délété (s).

29. Procédé selon la revendication 26, dans lequel la connexine n'existant pas naturellement est une connexine chimère, dans laquelle une région phosphorylée d'une queue carboxy d'une connexine phosphorylée est substituée par une connexine naturelle ou est ajoutée à celle-ci, laquelle n'est pas phosphorylée normalement.

30. Procédé selon la revendication 1, dans lequel la réponse de messager endogène est un taux accru d'adénosine monophosphate cyclique (AMPc), de calcium ou d'inositol triphosphate.

31. Procédé selon la revendication 1, dans lequel la réponse rapporteuse est détectée par une mesure du niveau d'expression d'une protéine exogène.

32. Procédé selon la revendication 31, dans lequel la protéine exogène est choisie dans le groupe constitué d'une luciférase de luciole, d'une luciférase bactérienne, de la phosphatase alcaline, de la protéine fluorescente verte (GFP) et d'une forme modifiée de la GFP, qui émet une fluorescence à une longueur d'onde différente de la GFP, de la bêta galactosidase et de la CAT.

33. Procédé pour identifier un composé à tester capable d'altérer la fonction de jonction communicante, comprenant les étapes consistant à :
(1) ajouter un stimulus exogène à une réaction d'essai, dans lequel la réaction d'essai comprend une cellule émettrice et une cellule receveuse, dans laquelle
(a) la cellule émettrice : (1) exprime une première connexine à la surface cellulaire, laquelle forme un premier connexon ; et (2) est capable de générer une réponse de messager endogène en réponse à un stimulus exogène ;
(b) la cellule receveuse : (1) exprime une deuxième connexine à la surface cellulaire, laquelle forme un deuxième connexon ; (2) n'est pas capable de générer une réponse de messager endogène à un stimulus exogène ; et (3) est capable de générer une réponse rapporteuse en réponse à la réponse de messager endogène générée par la cellule émettrice ;
(c) le premier connexon et le deuxième connexon forment une jonction communicante ;
(2) ajouter un composé à tester à la réaction d'essai ; et
(3) détecter la réponse rapporteuse ;
dans lequel la réponse rapporteuse est la transcription d'un deuxième gène rapporteur lié à un construit promoteur-rapporteur, contenant un élément répondant à l'AMPc (CRE), ou un gène rapporteur lié à un construit promoteur-rapporteur contenant un élément de réponse SRE ou TRE.

34. Procédé selon la revendication 33, dans lequel la première connexine est une connexine naturelle dans la cellule émettrice.

35. Procédé selon la revendication 34, dans lequel la connexine naturelle est une connexine de mammifère.

36. Procédé selon la revendication 35, dans lequel la connexine de mammifère est choisie dans le groupe constitué d'une connexine humaine, une connexine de primate, une connexine murine ou une connexine de rat.

37. Procédé selon la revendication 33, dans lequel la deuxième connexine est une connexine naturelle dans la cellule receveuse.

38. Procédé selon la revendication 37, dans lequel la connexine naturelle est une connexine de mammifère.

39. Procédé selon la revendication 38, dans lequel la connexine de mammifère est choisie dans le groupe constitué d'une connexine humaine, une connexine de primate, une connexine murine ou une connexine de rat.

40. Procédé selon la revendication 33, dans lequel la première connexine ou la deuxième connexine est choisie dans le groupe constitué d'une connexine naturelle de mammifère cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à dix différences d'acides aminés par rapport à une connexine naturelle.

41. Procédé selon la revendication 40, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à sept différences d'acides aminés par rapport à une connexine naturelle.

42. Procédé selon la revendication 41, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à quatre différences d'acides aminés par rapport à une connexine naturelle.

43. Procédé selon la revendication 42, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à trois différences d'acides aminés par rapport à une connexine naturelle.

44. Procédé selon la revendication 43, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

45. Procédé selon la revendication 44, dans lequel la première connexine ou la deuxième connexine est choisie dans le groupe constitué d'une connexine naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

46. Procédé selon la revendication 33, dans lequel la première connexine est une connexine non naturelle dans la cellule émettrice.

47. Procédé selon la revendication 46, dans lequel la connexine non naturelle est une connexine de mammifère.

48. Procédé selon la revendication 47, dans lequel la connexine de mammifère est choisie dans le groupe constitué d'une connexine humaine, une connexine de primate, une connexine murine ou une connexine de rat.

49. Procédé selon la revendication 48, dans lequel la deuxième connexine est une connexine non naturelle dans la cellule receveuse.

50. Procédé selon la revendication 49, dans lequel la connexine non naturelle est une connexine de mammifère.

51. Procédé selon la revendication 50, dans lequel la connexine de mammifère est choisie dans le groupe constitué d'une connexine humaine, une connexine de primate, une connexine murine ou une connexine de rat.

52. Procédé selon la revendication 33, dans lequel la première connexine ou la deuxième connexine est choisie dans le groupe constitué d'une connexine non naturelle de mammifère cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à dix différences d'acides aminés par rapport à une connexine naturelle.

53. Procédé selon la revendication 52, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à sept différences d'acides aminés par rapport à une connexine naturelle.

54. Procédé selon la revendication 53, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à quatre différences d'acides aminés par rapport à une connexine naturelle.

55. Procédé selon la revendication 54, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant d'une à trois différences d'acides aminés par rapport à une connexine naturelle.

56. Procédé selon la revendication 55, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

57. Procédé selon la revendication 56, dans lequel la première connexine ou la deuxième connexine est choisie dans le groupe constitué d'une connexine non naturelle cx26, cx30, cx30.3, cx31, cx31.1, cx32, cx33, cx36, cx37, cx40, cx43, cx45, cx46, cx50, cx57, CXN-311 ou un variant de celles-ci, comprenant une ou deux différences d'acides aminés par rapport à une connexine naturelle.

58. Procédé selon la revendication 33, dans lequel la première connexine ou la deuxième connexine est une connexine n'existant pas naturellement.

59. Procédé selon la revendication 58, dans lequel la connexine n'existant pas naturellement est une connexine chimère, dans laquelle un ou plusieurs acide(s) aminé(s) dans un ou plusieurs domaine(s) d'une connexine naturelle est (sont) substitué(s) par des acides aminés d'un domaine correspondant dans une connexine naturelle différente.

60. Procédé selon la revendication 59, dans lequel la connexine n'existant pas naturellement est une connexine chimère, dans laquelle un ou plusieurs acide(s) aminé(s) dans un ou plusieurs domaine(s) d'une connexine naturelle est (sont) substitué(s) par un acide aminé ayant une charge, une polarité ou une taille semblable ou bien est (sont) délété(s).

61. Procédé selon la revendication 59, dans lequel la connexine n'existant pas naturellement est une connexine chimère, dans laquelle une région phosphorylée d'une queue carboxy d'une connexine phosphorylée est substituée par une connexine naturelle ou est ajoutée à celle-ci, laquelle n'est pas phosphorylée normalement.

62. Procédé selon la revendication 33, dans lequel la réponse de messager endogène est un taux accru d'adénosine monophosphate cyclique (AMPc), de calcium ou d'inositol triphosphate.

63. Procédé selon la revendication 33, dans lequel la réponse rapporteuse est détectée par une mesure du niveau d'expression d'une protéine exogène.

64. Procédé selon la revendication 63, dans lequel la protéine exogène est choisie dans le groupe constitué d'une luciférase de luciole, d'une luciférase bactérienne, de la phosphatase alcaline, de la protéine fluorescente verte (GFP) et d'une forme modifiée de la GFP, qui émet une fluorescence à une longueur d'onde différente de la GFP, de la bêta galactosidase et de la CAT.


### SEQUENCE LISTING

<110> Barbier, Ann J
   Baron, Bruce M
<120> Gap Junction Permeability Assay
<130> USAV2001/0013
<140> 60/266,641
   <141> 2001-06-02
<160> 4
<170> PatentIn version 3.1
<210> 1
   <211> 8
   <212> DNA
   <213> Artificial
<400> 1
   tgacgtca
   8
<210> 2
   <211> 294
   <212> DNA
   <213> Artificial
<400> 2
   gagctccgat aagggctcgt tgacgtcacc aagaggcgat aagggctcgt tgacgtcacc
   60
   aagaggcgat aagggctcgt tgacgtcacc aagctccgat aagggctcgt tgacgtcacc 120
   aagctccgat aagggctcgt tgacgtcacc aagctccgat aagggctcgt tgacgtcacc 180
   aagctcactg tgtcgacgcg tgcaaggact ctatatatac agagggagct tcctagctgg 240
   gatattggag cagcaagagg ctgggaagcc atcacttacc ttgcactgag atct 294
<210> 3
   <211> 6365
   <212> DNA
   <213> Homo sapiens
<400> 3
   gacggatcgg gagatctccc gatcccctat ggtcgactct cagtacaatc tgctctgatg
   60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctagc 900
   gtttaaactt aagcttggta ccgagctcgg atccaagatg aggactctga acacctctgc 960
   catggacggg actgggctgg tggtggaaag ggacttctct gttcgtatcc tcacagcctg 1020
   tttcctgtcg ctgctcatcc tgtccacgct cctggggaac acgctggtct gtgctgccgt 1080
   tatcaggttc cgacacctgc ggtccaaggt gaccaacttc tttgtcatct ccttggctgt 1140
   gtcagatctc ttggtggccg tcctggtcat gccctggaag gcagtggctg agattgctgg 1200
   cttctggccc tttgggtcct tctgtaacat ctgggtggcc tttgacatca tgtgctccac 1260
   tgcatccatc ctcaacctct gtgtgatcag cgtggacagg tattgggcta tctccagccc 1320
   tttccggtat gagagaaaga tgacccccaa ggcagccttc atcctgatca gtgtggcatg 1380
   gaccttgtct gtactcatct ccttcatccc agtgcagctc agctggcaca aggcaaaacc 1440
   cacaagcccc tctgatggaa atgccacttc cctggctgag accatagaca actgtgactc 1500
   cagcctcagc aggacatatg ccatctcatc ctctgtaata agcttttaca tccctgtggc 1560
   catcatgatt gtcacctaca ccaggatcta caggattgct cagaaacaaa tacggcgcat 1620
   tgcggccttg gagagggcag cagtccacgc caagaattgc caaaccacca caggtaatgg 1680
   aaagcctgtc gaatgttctc aaccggaaag ttcttttaag atgtccttca aaagagaaac 1740
   taaagtcctg aagactctgt cggtgatcat gggtgtgttt gtgtgctgtt ggctaccttt 1800
   cttcatcttg aactgcattt tgcccttctg tgggtctggg gagacgcagc ccttctgcat 1860
   tgattccaac acctttgacg tgtttgtgtg gtttgggtgg gctaattcat ccttgaaccc 1920
   catcatttat gcctttaatg ctgattttcg gaaggcattt tcaaccctct taggatgcta 1980
   cagactttgc cctgcgacga ataatgccat agagacggtg agtatcaata acaatggggc 2040
   cgcgatgttt tccagccatc atgagccacg aggctccatc tccaaggagt gcaatctggt 2100
   ttacctgatc ccacatgctg tgggctcctc tgaggacctg aaaaaggagg aggcagctgg 2160
   catcgccaga cccttggaga agctgtcccc agccctatcg gtcatattgg actatgacac 2220
   tgacgtctct ctggagaaga tccaacccat cacacaaaac ggtcagcacc caacctgagg 2280
   atccactagt ccagtgtggt ggaattctgc agatatccag cacagtggcg gccgctcgag 2340
   tctagagggc ccgtttaaac ccgctgatca gcctcgactg tgccttctag ttgccagcca 2400
   tctgttgttt gcccctcccc cgtgccttcc ttgaccctgg aaggtgccac tcccactgtc 2460
   ctttcctaat aaaatgagga aattgcatcg cattgtctga gtaggtgtca ttctattctg 2520
   gggggtgggg tggggcagga cagcaagggg gaggattggg aagacaatag caggcatgct   2580
   ggggatgcgg tgggctctat ggcttctgag gcggaaagaa ccagctgggg ctctaggggg   2640
   tatccccacg cgccctgtag cggcgcatta agcgcggcgg gtgtggtggt tacgcgcagc   2700
   gtgaccgcta cacttgccag cgccctagcg cccgctcctt tcgctttctt cccttccttt   2760
   ctcgccacgt tcgccggctt tccccgtcaa gctctaaatc ggggcatccc tttagggttc   2820
   cgatttagtg ctttacggca cctcgacccc aaaaaacttg attagggtga tggttcacgt   2880
   agtgggccat cgccctgata gacggttttt cgccctttga cgttggagtc cacgttcttt   2940
   aatagtggac tcttgttcca aactggaaca acactcaacc ctatctcggt ctattctttt   3000
   gatttataag ggattttggg gatttcggcc tattggttaa aaaatgagct gatttaacaa   3060
   aaatttaacg cgaattaatt ctgtggaatg tgtgtcagtt agggtgtgga aagtccccag   3120
   gctccccagg caggcagaag tatgcaaagc atgcatctca attagtcagc aaccaggtgt   3180
   ggaaagtccc caggctcccc agcaggcaga agtatgcaaa gcatgcatct caattagtca   3240
   gcaaccatag tcccgcccct aactccgccc atcccgcccc taactccgcc cagttccgcc   3300
   cattctccgc cccatggctg actaattttt tttatttatg cagaggccga ggccgcctct   3360
   gcctctgagc tattccagaa gtagtgagga ggcttttttg gaggcctagg cttttgcaaa   3420
   aagctcccgg gagcttgtat atccattttc ggatctgatc agcacgtgtt gacaattaat   3480
   catcggcata gtatatcggc atagtataat acgacaaggt gaggaactaa accatggcca   3540
   agttgaccag tgccgttccg gtgctcaccg cgcgcgacgt cgccggagcg gtcgagttct   3600
   ggaccgaccg gctcgggttc tcccgggact tcgtggagga cgacttcgcc ggtgtggtcc   3660
   gggacgacgt gaccctgttc atcagcgcgg tccaggacca ggtggtgccg gacaacaccc   3720
   tggcctgggt gtgggtgcgc ggcctggacg agctgtacgc cgagtggtcg gaggtcgtgt   3780
   ccacgaactt ccgggacgcc tccgggccgg ccatgaccga gatcggcgag cagccgtggg   3840
   ggcgggagtt cgccctgcgc gacccggccg gcaactgcgt gcacttcgtg gccgaggagc   3900
   aggactgaca cgtgctacga gatttcgatt ccaccgccgc cttctatgaa aggttgggct   3960
   tcggaatcgt tttccgggac gccggctgga tgatcctcca gcgcggggat ctcatgctgg   4020
   agttcttcgc ccaccccaac ttgtttattg cagcttataa tggttacaaa taaagcaata   4080
   gcatcacaaa tttcacaaat aaagcatttt tttcactgca ttctagttgt ggtttgtcca   4140
   aactcatcaa tgtatcttat catgtctgta taccgtcgac ctctagctag agcttggcgt 4200
   aatcatggtc atagctgttt cctgtgtgaa attgttatcc gctcacaatt ccacacaaca 4260
   tacgagccgg aagcataaag tgtaaagcct ggggtgccta atgagtgagc taactcacat 4320
   taattgcgtt gcgctcactg cccgctttcc agtcgggaaa cctgtcgtgc cagctgcatt 4380
   aatgaatcgg ccaacgcgcg gggagaggcg gtttgcgtat tgggcgctct tccgcttcct 4440
   cgctcactga ctcgctgcgc tcggtcgttc ggctgcggcg agcggtatca gctcactcaa 4500
   aggcggtaat acggttatcc acagaatcag gggataacgc aggaaagaac atgtgagcaa 4560
   aaggccagca aaaggccagg aaccgtaaaa aggccgcgtt gctggcgttt ttccataggc 4620
   tccgcccccc tgacgagcat cacaaaaatc gacgctcaag tcagaggtgg cgaaacccga 4680
   caggactata aagataccag gcgtttcccc ctggaagctc cctcgtgcgc tctcctgttc 4740
   cgaccctgcc gcttaccgga tacctgtccg cctttctccc ttcgggaagc gtggcgcttt 4800
   ctcaatgctc acgctgtagg tatctcagtt cggtgtaggt cgttcgctcc aagctgggct 4860
   gtgtgcacga accccccgtt cagcccgacc gctgcgcctt atccggtaac tatcgtcttg 4920
   agtccaaccc ggtaagacac gacttatcgc cactggcagc agccactggt aacaggatta 4980
   gcagagcgag gtatgtaggc ggtgctacag agttcttgaa gtggtggcct aactacggct 5040
   acactagaag gacagtattt ggtatctgcg ctctgctgaa gccagttacc ttcggaaaaa 5100
   gagttggtag ctcttgatcc ggcaaacaaa ccaccgctgg tagcggtggt ttttttgttt 5160
   gcaagcagca gattacgcgc agaaaaaaag gatctcaaga agatcctttg atcttttcta 5220
   cggggtctga cgctcagtgg aacgaaaact cacgttaagg gattttggtc atgagattat 5280
   caaaaaggat cttcacctag atccttttaa attaaaaatg aagttttaaa tcaatctaaa 5340
   gtatatatga gtaaacttgg tctgacagtt accaatgctt aatcagtgag gcacctatct 5400
   cagcgatctg tctatttcgt tcatccatag ttgcctgact ccccgtcgtg tagataacta 5460
   cgatacggga gggcttacca tctggcccca gtgctgcaat gataccgcga gacccacgct 5520
   caccggctcc agatttatca gcaataaacc agccagccgg aagggccgag cgcagaagtg 5580
   gtcctgcaac tttatccgcc tccatccagt ctattaattg ttgccgggaa gctagagtaa 5640
   gtagttcgcc agttaatagt ttgcgcaacg ttgttgccat tgctacaggc atcgtggtgt 5700
   cacgctcgtc gtttggtatg gcttcattca gctccggttc ccaacgatca aggcgagtta 5760
   catgatcccc catgttgtgc aaaaaagcgg ttagctcctt cggtcctccg atcgttgtca 5820
   gaagtaagtt ggccgcagtg ttatcactca tggttatggc agcactgcat aattctctta 5880
   ctgtcatgcc atccgtaaga tgcttttctg tgactggtga gtactcaacc aagtcattct 5940
   gagaatagtg tatgcggcga ccgagttgct cttgcccggc gtcaatacgg gataataccg 6000
   cgccacatag cagaacttta aaagtgctca tcattggaaa acgttcttcg gggcgaaaac 6060
   tctcaaggat cttaccgctg ttgagatcca gttcgatgta acccactcgt gcacccaact 6120
   gatcttcagc atcttttact ttcaccagcg tttctgggtg agcaaaaaca ggaaggcaaa 6180
   atgccgcaaa aaagggaata agggcgacac ggaaatgttg aatactcata ctcttccttt 6240
   ttcaatatta ttgaagcatt tatcagggtt attgtctcat gagcggatac atatttgaat 6300
   gtatttagaa aaataaacaa ataggggttc cgcgcacatt tccccgaaaa gtgccacctg 6360
   acgtc 6365
<210> 4
   <211> 1341
   <212> DNA
   <213> Homo sapiens
<400> 4
   atgaggactc tgaacacctc tgccatggac gggactgggc tggtggtgga aagggacttc
   60
   tctgttcgta tcctcacagc ctgtttcctg tcgctgctca tcctgtccac gctcctgggg 120
   aacacgctgg tctgtgctgc cgttatcagg ttccgacacc tgcggtccaa ggtgaccaac 180
   ttctttgtca tctccttggc tgtgtcagat ctcttggtgg ccgtcctggt catgccctgg 240
   aaggcagtgg ctgagattgc tggcttctgg ccctttgggt ccttctgtaa catctgggtg 300
   gcctttgaca tcatgtgctc cactgcatcc atcctcaacc tctgtgtgat cagcgtggac 360
   aggtattggg ctatctccag ccctttccgg tatgagagaa agatgacccc caaggcagcc 420
   ttcatcctga tcagtgtggc atggaccttg tctgtactca tctccttcat cccagtgcag 480
   ctcagctggc acaaggcaaa acccacaagc ccctctgatg gaaatgccac ttccctggct 540
   gagaccatag acaactgtga ctccagcctc agcaggacat atgccatctc atcctctgta 600
   ataagctttt acatccctgt ggccatcatg attgtcacct acaccaggat ctacaggatt 660
   gctcagaaac aaatacggcg cattgcggcc ttggagaggg cagcagtcca cgccaagaat 720
   tgccaaacca ccacaggtaa tggaaagcct gtcgaatgtt ctcaaccgga aagttctttt     780
   aagatgtcct tcaaaagaga aactaaagtc ctgaagactc tgtcggtgat catgggtgtg     840
   tttgtgtgct gttggctacc tttcttcatc ttgaactgca ttttgccctt ctgtgggtct     900
   ggggagacgc agcccttctg cattgattcc aacacctttg acgtgtttgt gtggtttggg     960
   tgggctaatt catccttgaa ccccatcatt tatgccttta atgctgattt tcggaaggca    1020
   ttttcaaccc tcttaggatg ctacagactt tgccctgcga cgaataatgc catagagacg    1080
   gtgagtatca ataacaatgg ggccgcgatg ttttccagcc atcatgagcc acgaggctcc    1140
   atctccaagg agtgcaatct ggtttacctg atcccacatg ctgtgggctc ctctgaggac    1200
   ctgaaaaagg aggaggcagc tggcatcgcc agacccttgg agaagctgtc cccagcccta    1260
   tcggtcatat tggactatga cactgacgtc tctctggaga agatccaacc catcacacaa    1320
   aacggtcagc acccaacctg a                                              1341